# EUROPEAN PATENT SPECIFICATION

(11) **EP 3 522 862 B1**
(45) Date of publication and mention of the grant of the patent: **20.01.2021**
(21) Application number: 16781044.9
(22) Date of filing: 30.09.2016
(51) Int. Cl.: A61K 8/46, A61K 8/73, A61K 8/81, A61K 8/898, A61Q 5/02, A61Q 5/12

(54) **HAIR COSMETIC COMPOSITIONS CONTAINING THIOL-BASED COMPOUNDS AND METHODS FOR CLEANSING AND TREATING HAIR**
HAARKOSMETISCHE ZUSAMMENSETZUNGEN MIT THIOLBASIERTEN VERBINDUNGEN UND VERFAHREN ZUR REINIGUNG UND BEHANDLUNG VON HAAREN
COMPOSITIONS COSMÉTIQUES CAPILLAIRES CONTENANT DES COMPOSÉS À BASE DE THIOL ET PROCÉDÉS DE NETTOYAGE ET DE TRAITEMENT DES CHEVEUX

(43) Date of publication of application: 14.08.2019
(73) Proprietor: L'Oréal, 75008 Paris (FR); Gregolin, Marina Tavares, 21941-972 Rio de Janeiro, RJ (BR)
(72) Inventor: GREGOLIN, Marina Tavares, 21941-972 Rio de Janeiro - RJ (BR); DE MAGALHAES, Ana Paula Leme, 21941-972 Rio de Janeiro - RJ (BR); SATO, Bruno Maiko, 20090-010 Rio de Janeiro - RJ (BR); PETALI, Erika Alegrio Jarque, 21941-972 Rio de Janeiro - RJ (BR); MARTINS, Sintia Aguiar, 21941-972 Rio de Janeiro - RJ (BR); ALVES, Diego, 21941-972 Rio de Janeiro - RJ (BR)
(74) Representative: Casalonga
(86) International application number: PCT/BR2016/050244
(87) International publication number: WO 2018/058205

(56) References cited:
- WO-A1-03/086335
- DE-A1-102010 055 766
- DE-A1-102010 055 767

## Description

### FIELD OF THE INVENTION

The present application relates to cosmetic compositions for use on keratinous substrates, such as keratin fibers. In particular, it relates to compositions and methods for washing and/or cleansing hair and providing cosmetic and caring benefits to hair.

### BACKGROUND OF THE INVENTION

The quality and condition of hair is generally adversely affected by the action of external agents such as sunlight, wind, and humidity, and also by mechanical or chemical treatments, such as brushing, combing, shampooing, dyeing, bleaching, permanent-waving and/or relaxing. Hair is thus damaged by these various factors and may over time become dry, coarse or dull, especially in fragile areas, and more particularly at the ends leading to split ends.

In particular, cleansing compositions typically contain anionic surfactants. Anionic surfactants such as sodium lauryl sulfate have detergent properties and are highly effective at removing dirt and oil. However, anionic surfactants raise the cuticle of the hair for deep cleansing, but raised, rough cuticles also lead to frizziness of the hair. In addition, consumers generally find that products containing anionic surfactants can be too drying and damaging on frequent use, and, as a result, choose mild cleansing compositions which have low levels of anionic surfactants or do not contain anionic surfactants. However, such products may not always produce the desired amount of foaming and/or impart a clean feel to the hair. Consumers are therefore still in search of optimized cleansing compositions or cleansing/washing regimens and systems consisting of compositions with good or satisfactory foaming and effective cleansing qualities, and at the same time, provide adequate visual sleekness and smooth and conditioned feel to the hair, and in particular, control, or even eliminate frizziness, and also control or reduce the volume and the apparent mass of the head of hair.

Another area where consumers are always seeking better and unique products is in the area of hair cosmetic products designed to change the appearance, shape or configuration of hair. Examples of such hair cosmetic products are hair relaxers or hair straighteners which can relax or straighten curly or kinky hair, including wavy hair, or reduce/loosen curls while making the hair more manageable, untangled, more easily styled or shaped, and disciplined. Hair relaxers may either be applied in a hair salon by a professional or in the home by the individual consumer.

One type of composition that can be applied onto hair in order to change its shape and make it more manageable is an alkaline composition. Alkaline hair relaxing/straightening involves hydrolyzing the keratin of the hair with various alkaline agents, such as inorganic hydroxides, for instance sodium hydroxide, or organic hydroxides, such as guanidine hydroxide, or organic amines. Hair relaxing/straightening products that employ sodium hydroxide or potassium hydroxide are also called lye-based products and products that use other alkaline agents such as lithium hydroxide, calcium hydroxide, organic hydroxides and other non-hydroxide compounds, for example, organic amines, generally fall under the category of no-lye products. Still, it is desirable to find alternatives to the alkaline lye- and no-lye-based products and process described above which can damage the hair by weakening and/or causing dryness of the hair fibers WO03/086335 relates to the use of a hair care conditioning composition for restoring the elasticity of hair. The hair are restored and retain its elasticity by using a composition comprising lactic acid or a salt thereof. The composition may comprise thiolactate.

Thus, it is desirable for manufacturers of hair cosmetic products to formulate compositions with ingredients that can effectively cleanse the hair and at the same time, impart good styling/shaping or straightening benefits as well as good hair caring and manageability properties and other cosmetic properties such as conditioning, smooth feel, volume control/reduction, and frizz control/reduction.

However, the discovery of new compositions and processes for treating hair that have enhanced efficacy but impart less or minimal damage to hair, may pose challenges to manufacturers and formulators because the incorporation of new ingredients into the compositions may negatively impacting their performance, cosmetic attributes, texture, and formulation stability. In addition, the acidity/alkalinity and/or pH is an important consideration for these products. New processes for treating and changing the shape of hair may also impact the performance of the compositions, processing times and quality of use. Thus, manufacturers of such products continuously test the use of new raw materials and ingredients or new product forms and seek to re-formulate and create new products with the desired qualities, while still remaining stable and safe to use.

### SUMMARY OF THE INVENTION

The present invention is directed to a hair cosmetic agent composition, the composition comprising:
a) at least one cationic surfactant;
b) at least one amphoteric surfactant;
c) at least one thiol-based compound selected from thiolactic acid, their salts, and mixtures thereof;
d) at least one thickening agent;
e) at least one nonionic surfactant;
f) at least one cationic conditioning polymer;
g) optionally,at least one amino silicone; and
h) water; and
wherein the pH of the composition ranges from about 2 to less than 7.

The present invention is also directed to processes for treating the hair involving the application of the above-described composition onto hair.

### BRIEF DESCRIPTION OF THE DRAWINGS

Implementations of the present technology will now be described, by way of example only, with reference to the attached figures, wherein:
Fig. 1 provides images of hair swatches treated according to one application or treatment cycle including applying inventive or comparative shampoo formulas onto hair and heating the hair at a temperature of equal to or greater than 50ºC.
Fig. 2 provides images of hair swatches treated according to two application or treatment cycles including applying inventive or comparative shampoo formulas onto hair and heating the hair at a temperature of equal to or greater than 50ºC.
Fig. 3 provides images of hair swatches treated according to five application or treatment cycles including applying inventive or comparative shampoo formulas onto hair and heating the hair at a temperature of equal to or greater than 50ºC.

It should be understood that the various aspects are not limited to the arrangements and instrumentality shown in the drawings.

### DETAILED DESCRIPTION OF THE INVENTION

The present compositions effectively cleansing the hair and provide manageability properties to hair, including one or more of conditioning; straightening effects; frizz control; volume reduction or volume control; styling or shaping effects; curling effects; texlaxing effects; improvement or retention of curl definition; humidity resistance; cosmeticity to the feel; smooth feel; natural feel; less or reduced rough ends; and/or improvement of the appearance of hair.

"At least one" as used herein means one or more and thus includes individual components as well as mixtures/combinations.

"Keratinous substrates" as used herein, includes, but is not limited to keratin fibers such as hair and/or scalp on the human head.

"Conditioning" as used herein means imparting to one or more hair fibers at least one property chosen from combability, moisture-retentivity, luster, shine, and softness. The state of conditioning can be evaluated by any means known in the art, such as, for example, measuring, and comparing, the ease of combability of the treated hair and of the untreated hair in terms of combing work (gm-in), and consumer perception.

The term "treat" (and its grammatical variations) as used herein refers to the application of the compositions of the present disclosure onto the surface of keratinous substrates such as hair. The term 'treat" (and its grammatical variations) as used herein also refers to contacting keratinous substrates such as hair with the compositions of the present disclosure.

A "rinse-off" product refers to a composition such as a hair care composition that is rinsed and/or washed with water either after or during the application of the composition onto the keratinous substrate, and before drying and/or styling said keratinous substrate. At least a portion of the composition is removed from the keratinous substrate during the rinsing and/or washing.

The term "stable" as used herein means that the composition does not exhibit phase separation and/or crystallization.

"Volatile", as used herein, means having a flash point of less than about 100ºC.

"Non-volatile", as used herein, means having a flash point of greater than about 100ºC.

"Reducing agent" as used herein, means an agent capable of reducing the disulfide bonds of the hair.

"Active material" as used herein with respect to the percent amount of an ingredient or raw material, refers to 100% activity of the ingredient or raw material.

"Substituted," as used herein, means comprising one or more substituents. Non-limiting examples of substituents include atoms, such as oxygen atoms and nitrogen atoms, as well as functional groups, such as hydroxyl groups, ether groups, alkoxy groups, acyloxyalkyl groups, oxyalkylene groups, polyoxyalkylene groups, carboxylic acid groups, amine groups, acylamino groups, amide groups, halogen containing groups, ester groups, thiol groups, sulphonate groups, thiosulphate groups, siloxane groups, and polysiloxane groups. The substituent(s) may be further substituted.

"Polymers," as defined herein, include homopolymers and copolymers formed from at least two different types of monomers.

"INCI" is an abbreviation of International Nomenclature of Cosmetic Ingredients, which is a system of names provided by the International Nomenclature Committee of the Personal Care Products Council to describe personal care ingredients.

The compositions and methods of the present invention can comprise, consist of, or consist essentially of the essential elements and limitations of the invention described herein, as well as any additional or optional ingredients, components, or limitations described herein or otherwise useful.

Other than in the operating examples, or where otherwise indicated, all numbers expressing quantities of ingredients and/or reaction conditions are to be understood as being modified in all instances by the term "about," meaning within +/-5% of the indicated number.

All percentages, parts and ratios herein are based upon the total weight of the compositions of the present invention, unless otherwise indicated.

As used herein, all ranges provided are meant to include every specific range within, and combination of sub ranges between, the given ranges. Thus, a range from 1-5, includes specifically 1, 2, 3, 4 and 5, as well as sub ranges such as 2-5, 3-5, 2-3, 2-4, 1-4, etc.

In an embodiment, the present invention is directed to a hair cosmetic agent composition, the composition comprising:
(a) at least one cationic surfactant;
(b) at least one amphoteric surfactant;
(c) at least one thiol-based compound selected from thiolactic acid, their salts, and mixtures thereof;
(d) at least one thickening agent;
(e) at least one nonionic surfactant;
(f) at least one cationic conditioning polymer;
(g) optionally, at least one amino silicone; and
(h) water; and
wherein the pH of the composition ranges from about 2 to less than 7.

In one embodiment of the present invention, the at least one cationic surfactant is selected from optionally polyoxyalkenylated primary, secondary, tertiary fatty amine salts, quaternary ammonium salts, and mixtures thereof, and preferably from quaternary ammonium salts, and more preferably from cetrimonium chloride, behentrimonium chloride, behentrimonium methosulfate, dipalmitoylethylhydroxyethylmethylammonium methosulfate, and mixtures thereof.

In one embodiment of the present invention, the at least one cationic surfactant is present in an amount of from about 0.1% to about 10% by weight, preferably from about 0.5% to about 8% by weight, and most preferably from about 1% to about 5% by weight, relative to the total weight of the composition.

In one embodiment of the present invention, the at least one amphoteric surfactant is selected from (C8-C20)alkylbetaines, (C8-C20)alkylamido (C1-C6)alkylbetaines, sulfobetaines, (C8-C20)alkylsulfobetaines, (C8-C20)alkylamido(C1-C6)alkylsulfobetaines, (C8-C20)alkylamphoacetate, (C8-C20)alkylamphodiacetate, and their salts, and mixtures thereof.

In one embodiment of the present invention, the at least one amphoteric surfactant is selected from coco-betaine, cocamidopropylbetaine, sodium cocoamphoacetate, disodium cocoamphodiacetate, and mixtures thereof.

In one embodiment of the present invention, the at least one thiol-based compound is selected from thiolactic acid.

In one embodiment of the present invention, the at least one thiol-based compound is present in an amount of from about 0.5% to about 15% by weight, preferably from about 0.6% to about 14% by weight, more preferably from about 0.7% to about 12% by weight, even more preferably from about 0.8% to about 10% by weight, or from about 1% to about 8% by weight, based on the total weight of the composition.

In one embodiment of the present invention, the at least one thickening agent is selected from cellulose polymers, gums, modified or unmodified carboxyvinyl polymers, polyacrylamides, copolymers of acrylic acid and of acrylamide, sodium salts of polyhydroxycarboxylic acids, optionally crosslinked and/or neutralized 2-acrylamido-2-methylpropanesulphonic acid polymers and copolymers, polyacrylic acid/alkyl acrylate, glucans, modified or unmodified starches, silicas, and mixtures thereof.

In one embodiment of the present invention, the at least one thickening agent is selected from hydroxyethylcellulose (also known as HEC), hydroxymethylcellulose, methylhydroxyethylcellulose, hydroxypropylcellulose (also known as HPC), hydroxybutylcellulose, hydroxyethylmethylcellulose (also known as methyl hydroxyethylcellulose) and hydroxypropylmethylcellulose (also known as HPMC), cetyl hydroxyethylcellulose, hydroxypropyl guar gums, acrylate/C10 C30 alkyl acrylate copolymers, carbomers, polyacrylates, potato starch modified, and mixtures thereof.

In one embodiment of the present invention, the at least one nonionic surfactant is selected from fatty alcohols, alkoxylated fatty alcohols, alkyl(ether)phosphates, alkylpolyglucosides, fatty acid alkanolamides, and mixtures thereof.

In one embodiment of the present invention, the at least one nonionic surfactant is selected from linear, branched saturated/unsaturated fatty alcohols comprising from 6 to 60 carbon atoms and preferably from cetyl alcohol, stearyl alcohol, cetearyl alcohol (mixture of cetyl alcohol and stearyl alcohol), octyldodecanol, isostearyl alcohol, 2-hexyl decanol, palmityl alcohol, myristyl alcohol, stearyl alcohol, lauryl alcohol, oleic alcohol (or oleyl), linoleyl alcohol (or linoleyether), linolenic alcohol (or linolenyl) and undecylenic alcohol, and mixtures thereof, and more preferably from cetyl alcohol, stearyl alcohol, and cetearyl alcohol, PPG-5-Ceteth-10 phosphate, Oleth-3 phosphate, Oleth-10 phosphate, Ceteth-10 phosphate, a mixture of Ceteth-10 phosphate and Dicetyl phosphate, Dicetyl phosphate, Cetyl phosphate, Stearyl phosphate, laureth-7, laureth-9, trideceth-10, trideceth-12, C12-13 pareth-3, C12-13 pareth-23, C11-15 pareth-7, PPG-5 ceteth-20, PEG-55 Propylene Glycol Oleate, glycereth-26, decyl glucoside, cetearyl glucoside, decyl lauryl glucoside, stearyl glucoside, coco-glucoside, cocamide MIPA, and mixtures thereof.

In one embodiment of the present invention, the at least one cationic conditioning polymer is selected from cationic cellulose derivatives, cationic gum derivatives, polymer derivatives of diallyldimethyl ammonium chloride, polymer derivatives of methacrylamidopropyltrimethylammonium chloride, and mixtures thereof.

In one embodiment of the present invention, the at least one cationic conditioning polymer is selected from polyquaternium-7, polyquaternium-10, guar hydroxypropyltrimonium chloride, and mixtures thereof.

In one embodiment , the at least one amino silicone compound that may further comprise the compositions of the present invention is selected from amodimethicone, trideceth-9 PG amodimethicone, PEG-40/PPG-8 Methylaminopropyl/hydroxypropyl dimethicone copolymer, and mixtures thereof.

In one embodiment, the compositions of the present invention further comprise at least one neutralizing agent selected from organic amines, alkali metal hydroxides, alkali earth metal hydroxides, alkali metal carbonates, alkali metal phosphates, and mixtures thereof, and preferably selected from aminomethyl propanol, sodium hydroxide, potassium hydroxide, lithium hydroxide, aminomethyl propanediol, triisopropanol amine, dimethylstearylamine, dimethyl/tallowamine, lysine, ornithine, arginine, monoethanolamine, triethanolamine, calcium hydroxide, calcium bicarbonate, and mixtures thereof.

In one embodiment, the pH of the compositions of the present invention ranges from about pH 2.5 to about 6.5, or from about pH 3 to about 6, or from about pH 3 to about 5.2, such as from about pH 3 to about 5, or preferably from about pH 3 to about 4.8, or more preferably from about pH 3 to about 4.5, or even more preferably from about pH 3 to about 4, including all ranges and sub ranges therebetween.

In one embodiment, compositions of the of the present invention further comprise at least one additional component selected from organic solvents, plasticizers, opacifiers, plant/vegetable oils, hydrocarbons, lower alkanes, fragrance, preservatives, pH adjusters, plant extracts, salts, vitamins, sunscreens, colorants, and mixtures thereof.

In one embodiment, the hair cosmetic composition of the present invention comprises:
(a) at least one cationic surfactant selected from quaternary ammonium salts, and preferably from cetrimonium chloride, behentrimonium chloride, and mixtures thereof and present in an amount of from about 0,5% to about 8% by weight;
(b) at least one amphoteric surfactant selected from (C8-C20)alkylbetaines, preferably from coco betaine, cocamidopropylbetaine, cocoamphoacetate, cocoamphodiacetate, and their salts, and mixtures thereof and present in an amount of from about 0.8% to about 6% by weight;
(c) at least one thiol-based compound selected from thiolactic acid, their salts, and mixtures thereof and present in an amount of from about 0.7% to about 12% by weight;
(d) at least one thickening agent selected from hydroxyethylcellulose (also known as HEC), hydroxymethylcellulose, methylhydroxyethylcellulose, hydroxypropylcellulose (also known as HPC), hydroxybutylcellulose, hydroxyethylmethylcellulose (also known as methyl hydroxyethylcellulose) and hydroxypropylmethylcellulose (also known as HPMC), cetyl hydroxyethylcellulose, hydroxypropyl guar gums, acrylate/C10 C30 alkyl acrylate copolymers, carbomers, polyacrylates, potato starch modified, and mixtures thereof and present in an amount of from about 0.05% to about 1% by weight;
(e) at least one nonionic surfactant selected from fatty alcohols, alkoxylated fatty alcohols, alkyl(ether)phosphates, alkylpolyglucosides, fatty acid alkanolamides, and mixtures thereof, and present in an amount of from about 0.05% to about 10% by weight;
(f) at least one cationic conditioning polymer selected from cationic cellulose derivatives, cationic gum derivatives, polymer derivatives of diallyldimethyl ammonium chloride, polymer derivatives of methacrylamidopropyltrimethylammonium chloride, and mixtures thereof, and preferably from polyquaternium-7, polyquaternium-10, guar hydroxypropyltrimonium chloride, and mixtures thereof, and present in an amount of from about 0.05% to about 3% by weight;
(g) optionally, at least one silicone selected from amodimethicone, trideceth-9 PG amodimethicone, PEG-40/PPG-8 Methylaminopropyl/hydroxypropyl dimethicone copolymer, and mixtures thereof and present in an amount of from about 0,01% to about 5% by weight;
(h) water; and
(i) optionally, at least one neutralizing agent;
wherein the pH of the composition ranges from about 3 to about 5; all weights being relative to the total weight of the composition.

In one embodiment, the composition of the present invention is substantially free of anionic surfactants selected from sulfate surfactants, sulfonate surfactants, sarcosinate surfactants, and mixtures thereof.

In one embodiment, the composition of the present invention is a cleansing or shampoo composition.

In one embodiment, the present invention is directed to a method of treating hair, the method comprising a treatment cycle involving the steps of:
a) optionally, washing/rinsing the hair with a shampoo having a neutral pH and/or rinsing the hair with water, followed by allowing the hair to air dry or by drying the hair at a temperature ranging from room temperature up to about 100°C, while optionally applying a smoothing action on the hair;
b) applying the composition of any one of the preceding claims onto the hair;
c) allowing the composition to remain on the hair for a period of time ranging from about 1 to about 10 minutes or from about 1 to about 5 minutes;
d) rinsing the hair with water;
e) drying the hair at a temperature ranging from room temperature up to about 100ºC, while optionally applying a smoothing action on the hair;
f) passing a flat iron over the hair swatch at least once; and
g) washing/rinsing the hair with a shampoo having a neutral pH and/or rinsing the hair with water, followed by allowing the hair to air dry, while optionally applying a smoothing action on the hair.

In accordance with the present invention, Applicants have surprisingly and unexpectedly discovered that the above-described compositions which contain thiolactic acid in combination with certain surfactants provide cleansing benefits and other hair care benefits to hair fibers such as straightening or texlaxing effects, volume reduction, frizz control, curl reduction, manageability, discipline, cosmeticity, and smooth feel. While the foaming properties of the compositions of the present invention can be lighter or more mild than that of compositions containing sulfate-based anionic surfactants, the compositions of the present invention were still found to effectively cleanse the hair.

It was also surprisingly and unexpectedly discovered that the application of the compositions of the present invention when used in combination with the processes of the present invention, results in effectively cleansed hair and imparts/improves the cosmetic properties of hair fibers, in particular human hair fibers such as the hair, for example, in terms of manageability, straightening or texlaxing effects, curl reduction, volume reduction, frizz control, manageability, discipline, cosmeticity, and smooth feel.

The compositions described above may be used on any type of hair, for example, light or dark hair, straight or curly, natural hair, or hair that has undergone a cosmetic treatment such as permanent waving, dyeing, bleaching or relaxing.

In a preferred embodiment, the composition of the present invention is applied on curly, embrittled, and/or damaged hair.

Other subjects and characteristics, aspects and advantages of the invention will emerge even more clearly on reading the description and the example that follows.

### CATIONIC SURFACTANT

The composition according to the invention comprises one or more cationic surfactants.

Non-limiting examples of cationic surfactants useful in the invention include, for example, optionally polyoxyalkylenated primary, secondary or tertiary fatty amine salts, quaternary ammonium salts, and mixtures thereof.

Quaternary ammonium salts useful in the invention include, for example:
- quaternary ammonium salts having formula (Ia): 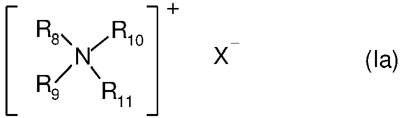 in which:
   the groups R8 to R11, which may be identical or different, represent a linear or branched aliphatic group containing from 1 to 30 carbon atoms, or an aromatic group such as aryl or alkylaryl, at least one of the groups R8 to R11 containing from 8 to 30 carbon atoms and preferably from 12 to 24 carbon atoms; it being possible for the aliphatic groups to comprise heteroatoms such as, in particular, oxygen, nitrogen, sulfur or halogens;

The aliphatic groups are chosen, for example, from C1-C30 alkyl, C1-C30 alkoxy, (C2-C6)polyoxyalkylene, C1-C30 alkylamide, (C12-C22)alkyl(C2C6)alkylamido, (C12-C22)alkyl acetate and C1-C30 hydroxyalkyl groups.

X- is an anion chosen from the group consisting of halides, phosphates, acetates, lactates, (C1-C4)alkyl sulfates, (C1-C4)alkylsulfonates and (C1-C4)alkylarylsulfonates.

Among the quaternary ammonium salts having formula (la), preference is given to tetraalkylammonium chlorides such as, for example, dialkyldimethylammonium or alkyltrimethylammonium chlorides in which the alkyl group comprises approximately from 12 to 22 carbon atoms, particularly behenyltrimethylammonium, distearyldimethylammonium, cetyltrimethylammonium, and benzyldimethylstearylammonium chlorides, or secondly, to palmitylamidopropyltrimethylammonium chloride or stearamidopropyldimethyl(myristyl acetate)-ammonium chloride, which is sold under the name Ceraphyl® 70 by the company Van Dyk.
- imidazoline quaternary ammonium salts having formula (IIa): 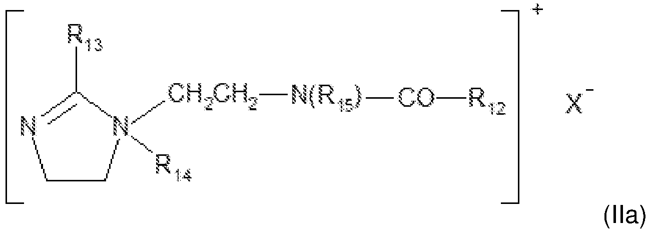 in which
   R12 represents an alkenyl or alkyl group comprising from 8 to 30 carbon atoms, for example fatty acid derivatives of tallow;
   R13 represents a hydrogen atom, a C1-C4 alkyl group or an alkenyl or alkyl group comprising from 8 to 30 carbon atoms;
   R14 represents a C1-C4 alkyl group;
   R15 represents a hydrogen atom or a C1-C4 alkyl group;
   X- is an anion chosen from the group consisting of halides, phosphates, acetates, lactates, (C1-C4)alkyl sulfates, (C1-C4)alkylsulfonates and (C1-C4)alkylarylsulfonates;
   R12 and R13 preferably denote a mixture of alkenyl or alkyl groups containing from 12 to 21 carbon atoms, for example fatty acid derivatives of tallow, R14 denotes a methyl group, and R15 denotes a hydrogen atom. A product of this kind is sold for example under the name Rewoquat® W 75 by the company Rewo.
- quaternary di- or triammonium salts having formula (Ilia): 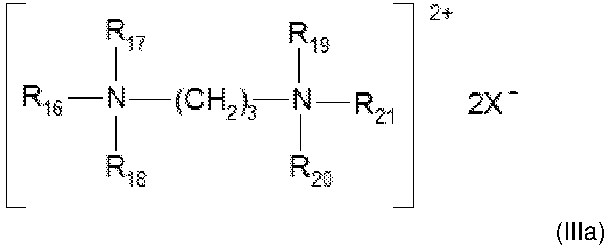 in which
   R16 denotes an alkyl group containing approximately from 16 to 30 carbon atoms, which is optionally hydroxylated and/or interrupted with one or more oxygen atoms, R17 is chosen from hydrogen or an alkyl group containing from 1 to 4 carbon atoms or a group -(CH2)3-N+(R16a)(R17a)(R18a);
   R16a, R17a, R18a, R18, R19, R20 and R21, which may be identical or different, are chosen from hydrogen and an alkyl group comprising from 1 to 4 carbon atoms; and
   X- is an anion chosen from the group consisting of halides, acetates, phosphates, nitrates, (C1-C4)alkyl sulfates, (C1-C4)alkylsulfonates and (C1-C4)alkylarylsulfonates, in particular methyl sulfate and ethyl sulfate.

Such compounds are, for example, Finquat® CT-P, sold by the company Finetex (Quaternium 89), and Finquat® CT, sold by the company Finetex (Quaternium 75),
- quaternary ammonium salts containing one or more ester functions having the following formula (IVa): 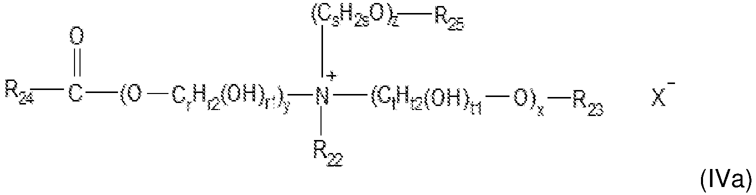 in which:
   R22 is chosen from C1-C6 alkyl groups and C1-C6 hydroxyalkyl or C1-C6 dihydroxyalkyl groups;
   R23 is chosen from the group R26-C(=O)-; hydrocarbon-based linear or branched, saturated or unsaturated C1-C22 groups R27; and a hydrogen atom;
   R25 is chosen from the group R28-C(=O)-; hydrocarbon-based linear or branched, saturated or unsaturated C1-C6 groups R29; and a hydrogen atom;
   R24, R26 and R28, which may be identical or different, are chosen from linear or branched, saturated or unsaturated C7-C21 hydrocarbon-based groups;
   r, s and t, which may be identical or different, are integers ranging from 2 to 6;
   r1 and t1, which may be identical or different, are equal to 0 or 1;
   r2 + r1 = 2 r and t1 + t2 = 2 t;
   y is an integer ranging from 1 to 10;
   x and z, which may be identical or different, are integers ranging from 0 to 10;
   X- is a simple or complex, organic or mineral anion;
   with the proviso that the sum x + y + z is from 1 to 15, that when x is 0 then R23 denotes R27, and that when z is 0 then R25 denotes R29.

The alkyl groups R22 may be linear or branched, and more particularly linear. Preferably, R22 denotes a methyl, ethyl, hydroxyethyl or dihydroxypropyl group, and more particularly a methyl or ethyl group.

Advantageously, the sum x + y + z is from 1 to 10.

When R23 is an R27 hydrocarbon group, it may be long and may have from 12 to 22 carbon atoms, or may be short and may have from 1 to 3 carbon atoms.

When R25 is an R29 hydrocarbon group, it preferably has 1 to 3 carbon atoms.

Advantageously, R24, R26 and R28, which are identical or different, are chosen from linear or branched, saturated or unsaturated C11-C21 hydrocarbon groups, and more particularly from linear or branched, saturated or unsaturated C11 - C21 alkyl and alkenyl groups.

Preferably, x and z, which may be identical or different, are equal to 0 or 1. Advantageously, y is equal to 1. Preferably, r, s and t, which may be identical or different, are equal to 2 or 3, and even more particularly are equal to 2.

The anion X- is preferably a halide, preferably chloride, bromide or iodide, a (C1-C4)alkyl sulfate, (C1-C4)alkyl sulfonate or (C1-C4)alkylaryl sulfonate. However, it is possible to use methanesulfonate, phosphate, nitrate, tosylate, an anion derived from an organic acid, such as acetate or lactate, or any other anion that is compatible with the ammonium comprising an ester function.

The anion X- is more particularly still chloride, methyl sulfate or ethyl sulfate.

Use is made more particularly, in the composition according to the invention, of the ammonium salts having formula (IVb) in which:
- R22 denotes a methyl or ethyl group,
- x and y are equal to 1,
- z is equal to 0 or 1,
- r, s and t are equal to 2,
- R23 is chosen from the group R26-C(=O)-; methyl groups, ethyl groups or hydrocarbon-based C14-C22 groups; and a hydrogen atom,
- R25 is chosen from the group R28-C(=O)-; and a hydrogen atom,
- R24, R26 and R28, which may be identical or different, are chosen from linear or branched, saturated or unsaturated C13-C17 hydrocarbon groups, and preferably from linear or branched, saturated or unsaturated C13-C17 alkyl and alkenyl groups.

The hydrocarbon-based groups are advantageously linear.

Among the compounds of formula (IVb), examples that may be mentioned include salts, in particular the chloride or methyl sulfate of diacyloxyethyldimethylammonium, diacyloxyethylhydroxyethylmethylammonium, monoacyloxyethyldihydroxyethylmethylammonium, triacyloxyethylmethylammonium or monoacyloxyethylhydroxyethyldimethylammonium, and mixtures thereof. The acyl groups preferably contain 14 to 18 carbon atoms and are obtained more particularly from a plant oil, such as palm oil or sunflower oil. When the compound contains several acyl groups, these groups may be identical or different.

These products are obtained, for example, by direct esterification of triethanolamine, triisopropanolamine, alkyldiethanolamine or alkyldiisopropanolamine, which are optionally oxyalkylenated, with fatty acids or with fatty acid mixtures of plant or animal origin, or by transesterification of the methyl esters thereof. This esterification is followed by a quaternization by means of an alkylating agent, such as an alkyl halide, preferably methyl or ethyl halide, a dialkyl sulfate, preferably methyl or ethyl sulfate, methyl methanesulfonate, methyl paratoluenesulfonate, glycol chlorohydrin or glycerol chlorohydrin. Such compounds are, for example, sold under the names Dehyquart® by the company Henkel, Stepanquat® by the company Stepan, Noxamium® by the company Ceca or Rewoquat® WE 18 by the company Rewo-Witco.

The composition according to the invention may contain, for example, a mixture of quaternary ammonium monoester, diester and triester salts with a weight majority of diester salts. Use may also be made of the ammonium salts containing at least one ester function that are described in patents US-A-4 874 554 and US-A-4 137 180. Use may also be made of behenoylhydroxypropyltrimethylammonium chloride, for example, sold by the company Kao under the name Quartamin BTC 131.

Preferably, the ammonium salts containing at least one ester function.

Preferably, the cationic surfactants are chosen from cetrimonim chloride (cetyltrimethylammonium salt), behentrimonim chloride (behenyltrimethylammonium salt), behentrimonium methosulfate, dipalmitoylethylhydroxyethylmethylammonium methosulfate, and mixtures thereof.

The composition according to the invention comprise the cationic surfactant in an amount ranging from about 0.1% to about 10% by weight, preferably from about 0.5% to about 8% by weight, and most preferably from about 0.7% to about 5% by weight, relative to the total weight of the composition, including all ranges and sub ranges therebetween.

In a particular embodiment, the amount of the at least one cationic surfactant is at about 0.1%, 0.125%, 0.15%, 0.2%, 0.25% 0.3%, 0.325%, 0.35%, 0.375%, 0.4%, 0.425%, 0.45%, 0.5%,0.55%, 0.6%, 0.65%, 0.7%, 0.75%, 0.8%, 0.85%, 0.9%, 0.95%, 1%, 1.25%, 1.5%, 1.75%, 2%, 2.25%, 2.4%, 2.5%, 2.75%, 3%, 3.25%, 3.5%, 3.75%, 4%, 4.25%, 4.5%, 4.75%, or 5% by weight, relative to the total weight of the composition.

### AMPHOTERIC SURFACTANT

The amphoteric surfactants of the present invention may be optionally quaternized secondary or tertiary aliphatic amine derivatives, in which the aliphatic group is a linear or branched chain comprising from 8 to 22 carbon atoms, said amine derivatives containing at least one anionic group, for instance a carboxylate, sulfonate, sulfate, phosphate or phosphonate group.

Mention may be made in particular of (C₈-C₂₀)alkylbetaines, (C₈-C₂₀)alkylamido (C₁-C₆)alkylbetaines, sulfobetaines, (C₈-C₂₀)alkylsulfobetaines, (C₈-C₂₀)alkylamido(C₁-C₆)alkylsulfobetaines, (C₈-C₂₀)alkylamphoacetate, (C₈-C₂₀)alkylamphodiacetate, and mixtures thereof.

Among the optionally quaternized secondary or tertiary aliphatic amine derivatives that may be used, mention may also be made of the products of respective structures (A1) and (A2) below:

(A1) Ra-CON(Z)CH₂-(CH₂)m-N+(Rb)(Rc)(CH₂COO-)

in which:
Ra represents a C₁₀-C₃₀ alkyl or alkenyl group derived from an acid Ra-COOH preferably present in hydrolysed coconut oil, a heptyl group, a nonyl group or an undecyl group,
Rb represents a β-hydroxyethyl group,
Rc represents a carboxymethyl group;
m is equal to 0, 1 or 2,
Z represents a hydrogen atom or a hydroxyethyl or carboxymethyl group;

(A2) Ra'-CON(Z)CH₂-(CH₂)m'-N(B)(B')

in which:
B represents -CH₂CH₂OX', with X' representing -CH₂-COOH, CH₂-COOZ', CH₂CH₂-COOH, -CH₂CH₂-COOZ', or a hydrogen atom,
B' represents -(CH₂)z-Y', with z = 1 or 2, and Y' representing COOH, COOZ', CH₂-CHOH-SO₃H or -CH₂-CHOH-SO₃Z',
m' is equal to 0, 1 or 2,
Z represents a hydrogen atom or a hydroxyethyl or carboxymethyl group,
Z' represents an ion resulting from an alkali or alkaline-earth metal, such as sodium, potassium or magnesium; an ammonium ion; or an ion resulting from an organic amine and in particular from an amino alcohol, such as monoethanola-mine, diethanolamine and triethanolamine, monoisopropanolamine, diisopropa-nolamine or triisopropanolamine, 2-amino-2-methyl-1-propanol, 2-amino-2-methyl-1,3-propanediol and tris(hydroxymethyl)aminomethane,
Ra' represents a C₁₀-C₃₀ alkyl or alkenyl group of an acid Ra'COOH preferably pre-sent in hydrolysed linseed oil or coconut oil, an alkyl group, in particular a C₁₇ alkyl group, and its iso form, or an unsaturated C₁₇ group.

Among the compounds corresponding to formula (A2) in which X' represents an hydrogen atom, mention may be made of compounds classified in the CTFA dictionary, under the names sodium cocoamphoacetate, sodium lauroamphoacetate, sodium caproamphoacetate and sodium capryloamphoacetate.

Other compounds corresponding to formula (A2) are disodium cocoamphodiace-tate, disodium lauroamphodiacetate, disodium caproamphodiacetate, disodium capryloamphodiacetate, disodium cocoamphodipropionate, disodium lauroam-phodipropionate, disodium caproamphodipropionate, disodium capryloamphodi-propionate, lauroamphodipropionic acid and cocoamphodipropionic acid.

Examples that may be mentioned include the cocoamphodiacetate sold by the company Rhodia under the trade name Miranol® C2M Concentrate, the sodium cocoamphoacetate sold under the trade name Miranol Ultra C 32 and the product sold by the company Chimex under the trade name CHIMEXANE HA.

Use may also be made of the compounds of formula (A3):

(A3) Ra"-NH-CH(Y")-(CH₂)n-C(O)-NH-(CH₂)n'-N(Rd)(Re)

in which:
- Ra" represents a C10-C30 alkyl or alkenyl group of an acid Ra"-C(O)OH preferably present in hydrolysed linseed oil or coconut oil;
- Y" represents the group -C(O)OH, -C(O)OZ", -CH₂-CH(OH)-SO₃H or the group CH₂-CH(OH)-SO₃-Z", with Z" representing a cationic counterion resulting from an alkali metal or alkaline-earth metal, such as sodium, an ammonium ion or an ion resulting from an organic amine;
- Rd and Re represent, independently of each other, a C₁-C₄ alkyl or hydroxyalkyl radical; and
- n and n' denote, independently of each other, an integer ranging from 1 to 3.

Among the compounds corresponding to formula (A3), mention may in particular be made of the compound classified in the CTFA dictionary under the name sodi-um diethylaminopropylcocoaspartamide, such as the one sold by the company Chimex under the name CHIMEXANE HB.

Preferably, the amphoteric surfactants are chosen from (C₈-C₂₀)alkylbetaines, (C₈-C₂₀)alkylamido(C₁-C₆)alkylbetaines, (C₈-C₂₀)alkylamphoacetates and (C₈-C₂₀)alkylamphodiacetates, and mixtures thereof.

In some embodiments, the at least one amphoteric surfactant of the present invention is chosen from (C₈-C₂₀)alkyl betaines, (C₈-C₂₀)alkylamido (C₁-C₆)alkylbetaines, (C₈-C₂₀)alkylamphoacetate, (C₈-C₂₀)alkylamphodiacetate, and their salts, and mixtures thereof.

Preferably, the amphoteric surfactants are chosen from (C₈-C₂₀)alkylbetaines such as the one known under the INCI names coco-betaine, (C₈-C₂₀)alkylamido(C₁-C₆)alkylbetaines such as the one known under the INCI name cocamidopropylbetaine, and mixtures thereof. Even more preferentially, the amphoteric surfactant is coco-betaine.

In one embodiment of the present invention, the at least one amphoteric surfactant is selected from coco-betaine, cocamidopropylbetaine, sodium cocoamphoacetate, disodium cocoamphodiacetate, and mixtures thereof.

In one embodiment of the present invention, the amount of amphoteric surfactants ranges from about 0.1% to about 10% by weight, preferably from about 0.5% to about 8% by weight, more preferably from about 0.8% to about 6% by weight, and even more preferably from about 1% to about 5% by weight, relative to the total weight of the composition, including all ranges and subranges therebetween.

In a particular embodiment, the amount of the at least one amphoteric surfactant is at about 1%, 1.25%, 1.5%, 1.55%, 1.6%, 1.65%, 1.7%, 1.75%, 1.8%, 1.85%, 1.9%, 1.95%, 2%, 2.05%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 3.0%, 3.25%, 3.5%, 3.75%, 4%, 4.25%, 4.5%, 4.75%, or 5% by weight, relative to the total weight of the composition.

### THIOL-BASED COMPOUNDS

The present invention employs at least one thiol-based compound selected from thiolactic acid, their salts, and mixtures thereof.

The at least one thiol-based compound of the present disclosure can be used in combination with other thiol-based compounds selected from thioglycolic acid, cysteine, cysteamine, homocystine, glutathione, thioglycerol, thiomalic acid, 2-mercaptopropionic acid, 3 -mercaptopropionic acid, thio diglyco I, 2-mercaptoethanol, dithiothreitol, thioxanthine, thiosalicylic acid, thiopropionic acid, lipoic acid, N-acetylcysteine, their salts thereof, and mixtures thereof.

The at least one thiol-based compound of the present disclosure can be also be used in combination with non-thiol based compounds such as alkali metal, alkaline-earth metal sulfites, hydrides or phosphines, and mixtures thereof.

In certain embodiments, the thiol-based compound used in the composition of the invention is thiolactic acid.

The at least one thiol-based compound can be employed in the compositions of the present invention in an amount of from about 0.5% to about 15% by weight, preferably from about 0.6% to about 14% by weight, more preferably from about 7% to about 12% by weight, more preferably from about 0.8% to about 10% by weight, or from about 1% to about 8% by weight, relative to the total weight of the composition, including all ranges and subranges therebetween.

In certain embodiments, the at least one thiol-based compound is selected from thiolactic acid and is employed in the composition of the present invention in an amount of about 1%, 1.25%, 1.5%, 1.75%, 2%, 2.25%, 2.5%, 2.75%, 3%, 3.25%, 3.5%, 3.75%, 4%, 4.25%, 4.5%, 4.75%, 5%, 5.25%, 5.5%, 5.75%, 6%, 6.25%, 6.5%, 6.75%, 7%, 7.25%, 7.5%, 7.75%, 8%, 8.25%, 8.5%, 8.75%, 9%, 9.5%, 10%, 10.5%, 11%, 11.5%, 12%, 13%, 14% by weight, based on the total weight of the composition.

### THICKENING AGENTS

The compositions according to the present disclosure may further comprise at least one thickening agent. Thickening agents are generally used to modify the viscosity and/or rheology of the composition. As used herein, the term "thickening agent" means compounds which, by their presence, increase the viscosity of the composition into which they are introduced by at least 20 cps, such as by at least 50 cps, at 25°C and at a shear rate of 1 s-1. The viscosity may be measured using a cone/plate viscometer, a Haake R600 rheometer, or the like.

The thickening agent may be referred to interchangeably herein as thickener or rheology modifier. Thickening agents are also sometimes referred to as gellifying agents and/or viscosity modifying agents.

In certain embodiments, the thickening agent may be chosen from those conventionally used in cosmetics, such as polymers of natural origin and synthetic polymers, for example, nonionic, anionic, cationic, amphiphilic, or amphoteric polymers, and other known rheology modifiers, such as cellulose-based thickeners.

In certain embodiments, the thickening agents can be an anionic thickening agent.

The anionic thickening agents may be chosen from hydrophilic thickeners. As used herein, the term "hydrophilic thickener" is meant to indicate that the thickening agent is soluble or dispersible in water. Non-limiting examples of hydrophilic thickeners include homopolymers or copolymers of acrylic or methacrylic acids or the salts thereof and the esters thereof, such as those sold under the tradenames Versicol F® or Versicol K® by the company Allied Colloid, or under the tradename Ultrahold 8® by the company Ciba-Geigy; polyacrylates and polymethacrylates such as copolymers of (meth)acrylic acid, copolymers of (meth)acrylic acid, methylacrylate and dimethyl meta-isopropenyl benzyl isocyanate of ethoxylated alcohols such as methylacrylate and dimethyl meta-isopropenyl benzyl isocyanate of ethoxylated alcohol (INCI name: Polyacrylate-3) sold under the tradename Viscophobe® DB 1000 from The Dow Chemical Company, those sold under the tradenames Lubrajel and Norgel by the company Guardian, or under the tradename Hispajel by the company Hispano Chimica; and polyacrylic acids of Synthalen® K type, copolymers of acrylic acid and of acrylamide sold in the form of the sodium salt thereof, such as those sold under the tradenames Reten® by Hercules, sodium polymethacrylate such as those sold under the tradename Darvan® 7 by the company Vanderbilt, and the sodium salts of polyhydroxycarboxylic acids such as those sold under the tradename Hydagen F® by the company Henkel, polyacrylic acid/alkyl acrylate copolymers of PemulenTM type, associative polymers, for instance PEG-150/stearyl alcohol/SMDI copolymer such those as sold under the tradename ACULYN™ 46 by the company Rohm & Haas, steareth-100/PEG-136/HDI copolymer such as those sold under the tradename Rheolate® FX 1100 by the company Elementis, and mixtures thereof.

As used herein, the term "copolymers" is intended to mean both copolymers obtained from two types of monomers and those obtained from more than two types of monomers, such as, for example, terpolymers obtained from three types of monomers. The chemical structure of the copolymers comprises at least one hydrophilic unit and at least one hydrophobic unit. The expression "hydrophobic unit" or "hydrophobic unit" is understood to mean a radical possessing a saturated or unsaturated and linear or branched hydrocarbon-based chain which comprises at least 8 carbon atoms, for example from 10 to 30 carbon atoms, as a further example from 12 to 30 carbon atoms, and as yet a further example from 18 to 30 carbon atoms.

In certain embodiments, the hydrophilic thickener may be chosen from anionic associative polymers. As used herein, the term "associative polymer" is intended to mean any polymer comprising in its structure at least one fatty chain and at least one hydrophilic portion.

In certain embodiments, the associative polymers may be chosen from polymers comprising at least one hydrophilic unit and at least one fatty-chain allyl ether unit; polymers in which the hydrophilic unit is constituted of an ethylenic unsaturated anionic monomer, such as a vinylcarboxylic acid, acrylic acid, methacrylic acid, or mixtures thereof; and polymers in which the fatty-chain allyl ether unit corresponds to the monomer of formula (I) below:

CH2 = C(R')CH2OBnR (I)

in which R' is chosen from H or CH3, B is an ethyleneoxy radical, n is zero or is chosen from an integer ranging from 1 to 100, and R is a hydrocarbon-based radical chosen from alkyl, arylalkyl, aryl, alkylaryl, or cycloalkyl radicals containing from 8 to 30 carbon atoms, from 10 to 24 carbon atoms, or from 12 to 18 carbon atoms. Exemplary and non-limiting polymers of this type are described and prepared, according to an emulsion polymerization process, in patent EP 0 216 479, incorporated by reference herein.

In certain embodiments, the associative anionic polymer may be chosen from anionic polymers comprising at least one hydrophilic unit of olefinic unsaturated carboxylic acid type, and at least one hydrophobic unit exclusively of (C10 C30)alkyl ester of unsaturated carboxylic acid type.

In certain embodiments, the at least one thickening agent is chosen from copolymers resulting from the polymerization of at least one monomer (a) chosen from carboxylic acids possessing α,β-ethylenically unsaturated groups or their esters, with at least one monomer (b) possessing ethylenically unsaturated groups and comprising a hydrophobic group. Such copolymers may exhibit emulsifying properties.

As used herein, the term "copolymers" is intended to mean both copolymers obtained from two types of monomers and those obtained from more than two types of monomers, such as, for example, terpolymers obtained from three types of monomers. The chemical structure of the copolymers comprises at least one hydrophilic unit and at least one hydrophobic unit. The expression "hydrophobic unit" or "hydrophobic unit" is understood to mean a radical possessing a saturated or unsaturated and linear or branched hydrocarbon-based chain which comprises at least 8 carbon atoms, for example from 10 to 30 carbon atoms, as a further example from 12 to 30 carbon atoms, and as yet a further example from 18 to 30 carbon atoms.

In certain embodiments, the thickening copolymer may be chosen from the copolymers resulting from the polymerization of:
(1) at least one monomer of formula (II):

   CH2=CH(R1)COOH (II)

   wherein R1 is chosen from H, CH3, or C2H5, providing acrylic acid, methacrylic acid, or ethacrylic acid monomers, and
(2) at least one monomer of (C10 C30)alkyl ester of unsaturated carboxylic acid type corresponding to the monomer of formula (III):

   CH2=CH(R2)COOR3 (III)

   wherein R2 is chosen from H, CH3, or C2H5, providing acrylate, methacrylate or ethacrylate units, and R3 denotes a C10 C30 alkyl radical, such as a C12 C22 alkyl radical.

In certain embodiments, the (C10 C30)alkyl esters of unsaturated carboxylic acids may be chosen from lauryl acrylate, stearyl acrylate, decyl acrylate, isodecyl acrylate, dodecyl acrylate or the corresponding methacrylates, such as lauryl methacrylate, stearyl methacrylate, decyl methacrylate, isodecyl methacrylate or dodecyl methacrylate, or mixtures thereof.

In certain embodiments, the crosslinked thickening polymer may be chosen from polymers resulting from the polymerization of a mixture of monomers comprising:
(1) acrylic acid,
(2) an ester of formula (III) described above, in which R2 is chosen from H or CH3, R3 denotes an alkyl radical having from 12 to 22 carbon atoms, and
(3) a crosslinking agent, which is a known copolymerizable polyethylenic unsaturated monomer, such as diallyl phthalate, allyl (meth)acrylate, divinylbenzene, (poly)ethylene glycol dimethacrylate and methylenebisacrylamide.

In various embodiments, the crosslinked thickening polymer may comprise from about 60 % to about 95 % by weight of acrylic acid (hydrophilic unit), from about 4 % to about 40 % by weight of C10 C30 alkyl acrylate (hydrophobic unit), and from about 0 % to about 6 % by weight of crosslinking polymerizable monomer. In further embodiments, the crosslinked thickening polymer may comprise from about 96 % to about 98 % by weight of acrylic acid (hydrophilic unit), from about 1 % to about 4 % by weight of C10 C30 alkyl acrylate (hydrophobic unit), and from about 0.1 % to 0.6 % by weight of crosslinking polymerizable monomer, such as those described above.

In some embodiments, the crosslinked thickening polymer may be chosen from acrylate/C10 C30 alkyl acrylate copolymers (INCI name: Acrylates/C10-30 Alkyl Acrylate Crosspolymer), such as the products sold under the tradenames Pemulen™ TR1, Pemulen™ TR2, Carbopol® 1382 and Carbopol® EDT 2020 by the company Lubrizol.

The anionic thickening agent may also be known as rheology modifiers such as acrylate- or acrylic-based polymers, carbomers, crosslinked homopolymers of acrylic acid or of acrylamidopropane-sulfonic acid, and crosslinked copolymers of (meth)acrylic acid and/or (C1-C6)alkyl esters.

In an embodiment, the anionic thickening agent is chosen from polyacrylate-3, commercially known under the trade name of Viscophobe DB-100 and sold by The Dow Chemical Company, carbomers, commercially known under the trade name of Carbopol polymers and sold by Lubrizol Advance Materials, Inc, acrylates/C10-30 alkyl acrylate crosspolymers, commercially known the trade names of Pemulen TR-1 and Pemulen TR-2 polymers and sold by Lubrizol Advance Materials, Inc, Acrylates/C10-30 Alkyl Acrylate Crosspolymer such as Carbopol® Ultrez 20 Polymer by and sold by Lubrizol Advance Materials, Inc,, AMP-acrylates/allyl methacrylate copolymer, commercially known under the trade name of Fixate G-100 polymer and sold by Lubrizol Advance Materials, Inc., Polyacrylate Crosspolymer-6 such as SepimaxTM Zen by the company Seppic, and a crosslinked methacrylic acid/ethyl acrylate copolymer, also known as an acrylates copolymer in aqueous dispersion, such as the slightly cross-linked, alkali-swellable acrylate polymer known by the INCI name acrylates copolymer and sold by Lubrizol, under the tradename CARBOPOL Aqua SF-1 as an aqueous dispersion comprising about 30 percent by weight of total solids or active material.

In various embodiments, the thickening agents may be chosen from hydrophilic thickeners, for example cellulose polymers and gums, modified or unmodified carboxyvinyl polymers, such as those sold under the tradename Carbopol® (CTFA name: carbomer) by the company Goodrich, polyacrylamides, copolymers of acrylic acid and of acrylamide sold in the form of the sodium salt thereof, such as those sold under the tradenames Reten® by Hercules, and the sodium salts of polyhydroxycarboxylic acids such as those sold under the tradename Hydagen F® by the company Henkel, optionally crosslinked and/or neutralized 2-acrylamido-2-methylpropanesulphonic acid polymers and copolymers, for instance poly(2-acrylamido-2-methylpropanesulphonic acid) such as those sold under the tradename Hostacerin® AMPS (CTFA name: ammonium polyacryldimethyltauramide) by the company Clariant, crosslinked anionic copolymers of acrylamide and of AMPS, e.g. in the form of a water-in-oil emulsion, such as those sold under the tradename Sepigel™ 305 (CTFA name: Polyacrylamide/C13-14 Isoparaffin/Laureth-7) and under the tradename Simugel™ 600 (CTFA name: Acrylamide/Sodium acryloyldimethyltaurate copolymer/Isohexadecane/Polysorbate 80) by the company Seppic, polyacrylic acid/alkyl acrylate copolymers of PemulenTM type, associative polymers, for instance PEG-150/stearyl alcohol/SMDI copolymer such those as sold under the tradename ACULYN™ 46 by the company Rohm & Haas, steareth-100/PEG-136/HDI copolymer such as those sold under the tradename Rheolate® FX 1100 by the company Elementis, and mixtures thereof.

In certain embodiments, the hydrophilic thickener may be chosen from associative polymers. As used herein, the term "associative polymer" is intended to mean any amphiphilic polymer comprising in its structure at least one fatty chain and at least one hydrophilic portion. As used herein, the term "amphiphilic polymer" means a polymer composed of hydrophilic and hydrophobic parts.

In certain embodiments, the associative polymers may be anionic, cationic, nonionic, or amphoteric. In certain embodiments, the associative polymers may be chosen from polymers comprising at least one hydrophilic unit and at least one fatty-chain allyl ether unit; polymers in which the hydrophilic unit is constituted of an ethylenic unsaturated anionic monomer, such as a vinylcarboxylic acid, acrylic acid, methacrylic acid, or mixtures thereof; and polymers in which the fatty-chain allyl ether unit corresponds to the monomer of formula (I) below:

CH2 = C(R')CH2OBnR (I)

in which R' is chosen from H or CH3, B is an ethyleneoxy radical, n is zero or is chosen from an integer ranging from 1 to 100, and R is a hydrocarbon-based radical chosen from alkyl, arylalkyl, aryl, alkylaryl, or cycloalkyl radicals containing from 8 to 30 carbon atoms, from 10 to 24 carbon atoms, or from 12 to 18 carbon atoms. Exemplary and non-limiting polymers of this type are described and prepared, according to an emulsion polymerization process, in patent EP 0 216 479, incorporated by reference herein.

In other embodiments, the associative cationic polymer may be chosen from quaternized cellulose derivatives and polyacrylates containing amine side groups.

In other embodiments, the non-ionic associative polymer may be chosen from celluloses modified with groups comprising at least one fatty chain, for instance hydroxyethyl celluloses modified with groups comprising at least one fatty chain, such as alkyl groups, e.g. C8-C22 alkyl groups, arylalkyl and alkylaryl groups; cetyl hydroxyethyl cellulose, also known under the tradename Natrosol® Plus (sold by the company Ashland), Bermocoll EHM 100 (sold by the company Berol Nobel), Amercell Polymer HM-1500® sold by the company Amerchol; hydroxyethylcellulose modified with a polyethylene glycol (15) nonylphenyl ether group, sold by the company Amerchol; celluloses modified with polyalkylene glycol alkylphenyl ether groups; guars such as hydroxypropyl guar, optionally modified with groups comprising at least one fatty chain such as an alkyl chain, for example Jaguar@ XC-95/3 (C14 alkyl chain, sold by the company Rhodia Chimie), Esaflor HM 22 (C22 alkyl chain, sold by the company Lamberti), RE210-18 (C14 alkyl chain) and RE205-1 (C20 alkyl chain, sold by the company Rhodia Chimie); copolymers of vinylpyrrolidone and of fatty-chain hydrophobic monomers, for instance Antaron® or Ganex® V216 (vinylpyrrolidone/hexadecene copolymers), Antaron® or Ganex® V220 (vinylpyrrolidone/eicosene copolymers) sold by the company I.S.P.; copolymers of C1-C6 alkyl methacrylates, acrylates or amphiphilic monomers comprising at least one fatty chain; or copolymers of hydrophilic methacrylates, acrylates or hydrophobic monomers comprising at least one fatty chain, for instance the polyethylene glycol methacrylate/lauryl methacrylate copolymer.

Associative polyurethanes may also be chosen in various embodiments. As used herein, "associative polyurethanes" are nonionic block copolymers comprising in the chain both hydrophilic blocks usually of polyoxyethylene nature, and hydrophobic blocks that may be aliphatic sequences alone and/or cycloaliphatic and/or aromatic sequences. Associative polyurethanes comprise at least two hydrocarbon-based lipophilic chains containing from C6 to C30 carbon atoms, separated by a hydrophilic block, the hydrocarbon-based chains optionally being pendent chains or chains at the end of a hydrophilic block. For example, it is possible for one or more pendent chains to be provided. In addition, the polymer may comprise a hydrocarbon-based chain at one or both ends of a hydrophilic block. The associative polyurethanes may be arranged in triblock or multiblock form. The hydrophobic blocks may thus be at each end of the chain (for example, triblock copolymer with a hydrophilic central block) or distributed both at the ends and within the chain (for example, multiblock copolymer). These polymers may also be graft polymers or starburst polymers. In one embodiment, the associative polyurethanes may be triblock copolymers in which the hydrophilic block is a polyoxyethylene chain containing from 50 to 1000 oxyethylene groups.

In other embodiments, associative polymers of the polyurethane polyether type that may be used include the polymer C16-OE120-C16 from Servo Delden (under the tradename SER AD FX1100), which is a molecule containing a urethane function and having a weight-average molecular weight of 1300, OE being an oxyethylene unit; Nuvis® FX 1100 (European and US INCI name "Steareth-100/PEG-136/HMDI Copolymer" sold by the company Elementis Specialties); Acrysol RM 184® (sold by the company Rohm and Haas); Elfacos® T210® (C12-C14 alkyl chain); Elfacos® T212® (C18 alkyl chain) sold by the company Akzo; Rheolate® 205 containing a urea function, sold by the company Rheox; RHEOLATE® 208 or 204, or RHEOLATE® FX1100 sold by the company Elementis; or DW 1206B sold by the company Rohm & Haas containing a C20 alkyl chain with a urethane bond, sold at a solids content of 20% in water.

In further embodiments, solutions or dispersions of these polymers, especially in water or in aqueous-alcoholic medium, may be chosen. Examples of such polymers include SER AD FX1010, SER AD FX1035, and SER AD 1070 from the company Servo Delden, and Rheolate® 255, Rheolate® 278, and Rheolate® 244 sold by Rheox. Further examples include the products Aculyn™ 46, DW 1206F, and DW 1206J, Acrysol RM 184 or Acrysol 44 from the company Rohm & Haas, and Borchi® Gel LW 44 from the company Borchers.

In further embodiments, the thickening agent may be chosen from nonionic homopolymers or copolymers containing ethylenically unsaturated monomers of the amide type, for example, the polyacrylamide products sold under the tradenames Cyanamer® P250 by the company CYTEC.

In further embodiments, the thickening agent chosen from polymers of natural origin may include thickening polymers comprising at least one sugar unit, for instance nonionic guar gums, optionally modified with C1-C6 hydroxyalkyl groups; biopolysaccharide gums of microbial origin, such as scleroglucan gum (also known as sclerotium gum) or xanthan gum; gums derived from plant exudates, such as gum arabic, ghatti gum, karaya gum, gum tragacanth, carrageenan gum, agar gum, carob gum, ceratonia siliqua gum or cyamopsis tetragonoloba (guar) gum; pectins; alginates; starches; hydroxy(C1-C6)alkylcelluloses; or carboxy(C1-C6)alkylcelluloses.

In certain embodiments, the nonionic, unmodified guar gums may be chosen from Guargel D/15 sold by the company Noveon, Vidogum GH 175 sold by the company Unipectine, Meypro-Guar 50 sold by the company Meyhall, or Jaguar@ C sold by the company Rhodia Chimie. In other embodiments, the nonionic modified guar gums may be chosen from Jaguar@ HP8, HP60, HP120, DC 293 and HP 105 sold by the companies Meyhall and Rhodia Chimie or GalactasolTM 4H4FD2 sold by the company Ashland.

In other embodiments, the thickening agents may be chosen from scleroglucans, for example, Actigum™ CS from Sanofi Bio Industries; Amigel® sold by the company Alban Muller International; xanthan gums, for instance Keltrol®, Keltrol® T, Keltrol® Tf, Keltrol® Bt, Keltrol® Rd, and Keltrol® Cg sold by the company CP Kelco, Rhodicare® S and Rhodicare® H sold by the company Rhodia Chimie; starch derivatives, for instance Primogel® sold by the company Avebe; hydroxyethylcelluloses such as Cellosize® QP3L, QP4400H, QP30000H, HEC30000A and Polymer PCG10 sold by the company Amerchol, Natrosol™ 250HHR, 250MR, 250M, 250HHXR, 250HHX, 250HR, and 250 HX, sold by the company Hercules, or Tylose® H1000 sold by the company Hoechst; hydroxypropylcelluloses, for instance KlucelTM EF, H, LHF, MF, and G, sold by the company Ashland; carboxymethylcelluloses, for instance Blanose® 7M8/SF, refined 7M, 7LF, 7MF, 9M31F, 12M31XP, 12M31P, 9M31XF, 7H, 7M31, and 7H3SXF, sold by the company Ashland, Aquasorb® A500 sold by the company Hercules, Ambergum® 1221 sold by the company Hercules, Cellogen® HP810A and HP6HS9 sold by the company Montello and Primellose® sold by the company Avebe.

In other embodiments, the modified nonionic guar gums may, for example, be modified with C1-C6 hydroxyalkyl groups. Such hydroxyalkyl groups may be chosen from hydroxymethyl, hydroxyethyl, hydroxypropyl, and hydroxybutyl groups.

In certain embodiments, guar gums may be prepared by reacting the corresponding alkylene oxides, such as for example propylene oxides, with guar gum so as to obtain a guar gum modified with hydroxypropyl groups. The hydroxyalkylation ratio, which corresponds to the number of alkylene oxide molecules consumed to the number of free hydroxyl functional groups present on the guar gum, may in certain embodiments range from about 0.4 to about 1.2.

Examples of nonionic guar gums, optionally modified with hydroxyalkyl groups, include those sold under the tradenames Jaguar@ HP8, Jaguar@ HP60, Jaguar@ HP120, Jaguar@ DC 293, and Jaguar@ HP 105 by the company Rhodia Chimie, and under the tradename Galactasol™ 4H4FD2 by the company Ashland.

In other embodiments, the guar gum may be chosen from those modified with a quaternary ammonium group, such as guar hydroxypropyltrimonium chloride, also sold under the tradename Jaguar@ C-13S by the company Rhodia Chimie.

In other embodiments, the ceulloses may be chosen from hydroxyethylcelluloses and hydroxypropylcelluloses, such as those sold under the tradenames KlucelTM EF, KlucelTM H, KlucelTM LHF, KlucelTM MF, KlucelTM G, by the company Ashland and under the tradename CellosizeTM PCG-10 by the company Amerchol.

In other embodiments, non-limiting thickening polysaccharides may be chosen from glucans; modified or unmodified starches such as those derived, for example, from cereals such as wheat, corn or rice, vegetables such as golden pea, or tubers such as potato or cassava; amylose, amylopectin, glycogen, dextrans, celluloses or derivatives thereof (methylcelluloses, hydroxyalkylcelluloses, ethylhydroxyethylcelluloses), mannans, xylans, lignins, arabans, galactans, galacturonans, chitin, chitosans, glucoronoxylans, arabinoxylans, xyloglucans, glucomannans, pectic acids or pectins, arabinogalactans, carrageenans, agars, gums arabic, gums tragacanth, Ghatti gums, Karaya gums, carob gums, galactomannans such as guar gums and their nonionic derivatives such as hydroxypropylguar, or mixtures thereof.

In other embodiments, the thickening agent may be chosen from silicas or hydrophobic silicas, such as those described in EP-A-898960, incorporated by reference herein. Examples of such silicas include those sold under the tradename Aerosil® R812 by the company Degussa, CAB-O-SIL® TS-530, CAB-O-SIL® TS-610, CAB-O-SIL® TS-720 by the company Cabot, or Aerosil® R972 and Aerosil® R974 by the company Degussa; clays, such as montmorillonite; modified clays such as the bentones, for example, stearalkonium hectorite, stearalkonium bentonite; or polysaccharide alkyl ethers, optionally with the alkyl group having from 1 to 24 carbon atoms, for example from 1 to 10 carbon atoms, from 1 to 6 carbon atoms, or from 1 to 3 carbon atoms, such as those described in document EP-A-898958, incorporated by reference herein.

In certain embodiments, when an anionic thickening agent is used, it is generally neutralized before being included in, or as it is added to the compositions of the disclosure. Such an anionic thickening agent may be neutralized by employing traditional neutralizing agents such as alkanolamines, for example, monoethanolamine and diethanolamine; aminomethyl propanol; basic amino acids, for example arginine and lysine; or ammonium compounds and their salts. The anionic thickening agent may also be neutralized by a latex polyurethane polymer having at least one free amino group.

In one embodiment of the present invention, the at least one thickening agent is selected from cellulose polymers, gums, modified or unmodified carboxyvinyl polymers, polyacrylamides, copolymers of acrylic acid and of acrylamide, sodium salts of polyhydroxycarboxylic acids, optionally crosslinked and/or neutralized 2-acrylamido-2-methylpropanesulphonic acid polymers and copolymers, polyacrylic acid/alkyl acrylate, glucans, modified or unmodified starches, silicas, and mixtures thereof.

In one embodiment, the thickening agent in the compositions of the present invention comprises a starch derivative chosen from the compounds of the following formulae: 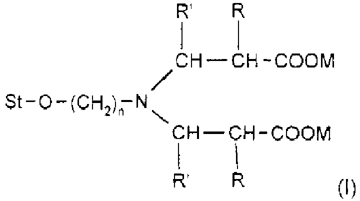 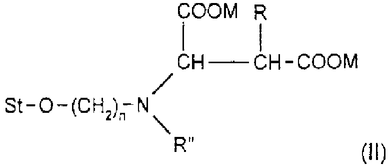 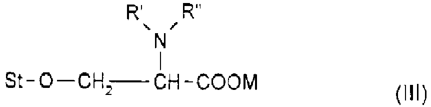 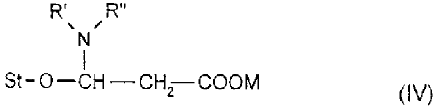 in which formulae:
St-O represents a starch molecule,
R, which may be identical or different, represents a hydrogen atom or a methyl radical,
R', which may be identical or different, represents a hydrogen atom, a methyl radical or a -COOH group,
n is an integer equal to 2 or 3,
M, which may be identical or different, denotes a hydrogen atom, an alkali metal or alkaline-earth metal such as Na, K or Li, NH₄, a quaternary ammonium or an organic amine,
R" represents a hydrogen atom or an alkyl radical containing from 1 to 18 carbon atoms.

The starch molecules may be derived from any plant sources of starch such as, in particular, corn, potato, oat, rice, tapioca, sorghum, barley or wheat. The starch hydrolysates mentioned above may also be used. The starch is preferably derived from potato.

The starches of formula (I) or (II) are used in particular. Starches modified with 2-chloroethylaminodipropionic acid, i.e. the starches of formula (I) or (II) in which R, R' and R" represent a hydrogen atom and n is equal to 2, are used more preferentially.

A preferred starch derivative is known under the INCI name potato starch modified sold under the tradename STRUCTURE® SOLANACE by AkzoNobel.

The starch derivative may be present in the compositions of the invention in an amount ranging from about 0.05% to about 3%, preferably from about 0.1% to about 2.5%, and most preferably from about 0.1% to about 2% by weight relative to the total weight of the composition.

In one embodiment of the present invention, the at least one thickening agent is selected from hydroxyethylcellulose (also known as HEC), hydroxymethylcellulose, methylhydroxyethylcellulose, hydroxypropylcellulose (also known as HPC), hydroxybutylcellulose, hydroxyethylmethylcellulose (also known as methyl hydroxyethylcellulose) and hydroxypropylmethylcellulose (also known as HPMC), cetyl hydroxyethylcellulose, hydroxypropyl guar gums, acrylate/C10 C30 alkyl acrylate copolymers, carbomers, polyacrylates, potato starch modified, and mixtures thereof.

In one embodiment of the present invention, the at least one thickening agent comprises hydroxyethylcellulose, cetyl hydroxyethylcellulose, and mixtures thereof.

In one embodiment of the present invention, the at least one thickening agent comprises hydroxyethylcellulose, cetyl hydroxyethylcellulose, potato starch modified and mixtures thereof.

In one embodiment of the present invention, the at least one thickening agent is present in a total amount of from about 0.01% to about 5% by weight, preferably from about 0.05% to about 3% by weight, and most preferably from about 0.1% to about 2% by weight, relative to the total weight of the composition, including all ranges and subranges therebetween.

In various embodiments, the thickening agent may be present in an amount of about 0.01%, 0.02%, 0.03%, 0.04%, 0.05%, 0.06%, 0.07%, 0.075%, 0.08%, 0.09%, 0.1%, 0.125%, 0.15%, 0.2%, 0.25% 0.3%, 0.325%, 0.35%, 0.375%, 0.4%, 0.425%, 0.45%, 0.5%,0.55%, 0.6%, 0.65%, 0.7%, 0.75%, 0.8%, 0.85%, 0.9%, 0.95%, 1%, 1.25%, 1.5%, 1.75%, or 2% by weight, relative to the total weight of the composition.

### NONIONIC SURFACTANTS

In one embodiment, the compositions of the present invention comprise at least one nonionic surfactant selected from fatty alcohols, alkoxylated fatty alcohols, alkyl(ether)phosphates, alkylpolyglucosides, fatty acid alkanolamides, and mixtures thereof.

### Fatty Alcohols

The fatty alcohols correspond to linear, branched saturated/unsaturated fatty alcohols comprising from 6 to 60 carbon atoms and preferably correspond to the formula R-OH in which R is a saturated or unsaturated, linear or branched hydrocarbon-based radical, comprising 6 to 60 carbon atoms, or from 10 to 50 carbon atoms, or from 12 to 24 carbon atoms, or from 10 to 22 carbon atoms optionally comprising one or more OH groups.

The saturated fatty alcohols are preferably branched and can be in liquid form. They can optionally comprise, in their structure, at least one aromatic or non-aromatic ring. They are preferably acyclic.

The unsaturated fatty alcohols exhibit, in their structure, at least one double or triple bond and preferably one or more double bonds. When several double bonds are present, there are preferably 2 or 3 of them and they can be conjugated or unconjugated. These unsaturated fatty alcohols can be linear or branched. They can optionally comprise, in their structure, at least one aromatic or non-aromatic ring. They are preferably acyclic.

Liquid fatty alcohols may be selected, for example, from octyldodecanol, 2-butyloctanol, 2-hexyldecanol, 2-undecylpentadecanol, oleyl alcohol, linoleyl alcohol, isostearyl alcohol, undecylenyl alcohol, linolenyl alcohol and mixtures thereof.

The fatty alcohols of the invention may be in solid form and may be non-oxyalkylenated and/or non-glycerolated. These fatty alcohols may be constituents of animal or plant waxes.

The solid fatty alcohol may represent a mixture of fatty alcohols, which means that several species of fatty alcohol may coexist, in the form of a mixture, in a commercial product. One example of such a commercial product is cetearyl alcohol, a mixture of cetyl alcohol and stearyl alcohol, commercially available under the trade name of LANETTE-O from the company BASF. Cetyl alcohol may also be commercially available under the tradename of LANETTE 16 from the company BASF.

In an embodiment, the solid fatty alcohols of the present invention may be chosen from myristyl alcohol, cetyl alcohol, stearyl alcohol, cetearyl alcohol, and mixtures thereof, octyldodecanol, 2-butyloctanol, 2-hexyldecanol, 2-undecylpentadecanol, oleic alcohol, linoleic alcohol, behenyl alcohol, and mixtures thereof.

Other suitable examples of the solid fatty alcohol of the present invention include branched solid fatty alcohols chosen from 2-dodecylhexadecanol, 2-tetradecyl-1-octadecanol, 2-tetradecyl-1-eicosanol, 2-hexadecyl-1-octadecanol and 2-hexadecyl-1-eicosanol, and mixtures thereof.

In embodiment of the present invention, the fatty alcohol comprises cetyl alcohol and stearyl alcohol or cetearyl alcohol.

In embodiment of the present invention, the fatty alcohol is chosen from cetyl alcohol, stearyl alcohol, cetearyl alcohol, and mixtures thereof.

### Alkoxylated fatty alcohols

"Alkoxylated fatty alcohol" as used herein means a compound having at least one fatty portion (8 carbon atoms or more) and at least one alkoxylated portion (-(CH₂)ₙO-, where n is an integer from 1 to 5, preferably 2 to 3). According to particularly preferred embodiments, the alkoxylated fatty alcohols of the present invention can be used as non-ionic surfactants, if desired. In this regard, the alkoxylated fatty alcohols of the present invention preferably have an HLB (hydrophilic-lipophilic balance) value from 1-20, including all ranges and subranges therebetween, with HLB values ranging from 1 to 5 (particularly 3 to 5) or from 15-20 (particularly 16 to 18) being most preferred. Preferably, the alkoxylated fatty alcohol is chosen from ethoxylated fatty alcohols, propoxylated fatty alcohols, and mixtures thereof.

Preferably, the alkoxylated fatty alcohol can be chosen from di-alkyl, tri-alkyl- and combinations of di-alkyl and tri-alkyl substituted ethoxylated polymers. They can also be chosen from mono-alkyl, di-alkyl, tri-alkyl, tetra-alkyl substituted alkyl ethoxylated polymers and all combinations thereof. The alkyl group can be saturated or unsaturated, branched or linear and contain a number of carbon atoms preferably from about 12 carbon atoms to about 50 carbon atoms, including all ranges and subranges therebetween, for example, 20 to 40 carbon atoms, 22 to 24 carbon atoms, 30 to 50 carbon atoms, and 40 to 60 carbon atoms. Most preferably, the fatty portion contains a mixture of compounds of varying carbon atoms such as, for example, C20-C40 compounds, C22-C24 compounds, C30-050 compounds, and C40-C60 compounds.

Preferably, the alkoxylated portion of the alkoxylated fatty alcohols of the present invention contain 2 or more alkoxylation units, preferably from 10 to 200 alkoxylation units, preferably from 20 to 150 alkoxylation units, and preferably from 25 to 100 alkoxylation units, including all ranges and subranges therebetween. Also preferably, the alkoxylation units contain 2 carbon atoms (ethoxylation units) and/or 3 carbon atoms (propoxylation units).

The amount of alkoxylation can also be determined by the percent by weight of the alkoxylated portion with respect to the total weight of the compound. Suitable weight percentages of the alkoxylated portion with respect to the total weight of the compound include, but are not limited to, 10 percent to 95 percent, preferably 20 percent to 90 percent, including all ranges and subranges therebetween with 75 percent to 90 percent (particularly 80 percent to 90 percent) or 20 percent to 50 percent being preferred.

Preferably, the alkoxylated fatty alcohols of the present invention have a number average molecular weight (Mn) greater than 500, preferably from 500 to 5,000, including all ranges and subranges therebetween such as, for example, Mn of 500 to 1250 or an Mn of 2,000 to 5,000.

Suitable examples of alkoxylated fatty alcohols include: laureth-3, laureth-7, laureth-9, laureth-12, laureth∼23, ceteth-10, steareth-10, steareth-2, steareth-100, beheneth-5, beheneth-5, beheneth-10, oleth-10, Pareth alcohols, trideceth-10, trideceth-12, C12-13 pareth-3, C12-13 pareth-23, C11-15 pareth-7, PPG-5 ceteth-20, PEG-55 Propylene Glycol Oleate, glycereth-26 (PEG-26 Glyceryl Ether), PEG 120 methyl glucose dioleate, PEG 120 methyl glucose trioleate, PEG 150 pentaerythrityl tetrastearate, and mixtures thereof.

### Alkyl(ether)phosphates

Suitable alkyl(ether)phosphates include, but are not limited to, alkoxylated alkyl phosphate esters and alkyl phosphate esters corresponding to a mono-ester of formula (I) and salts thereof:

RO[CH₂O]ᵤ[(CH₂)ₓCH(R')(CH₂)_{y}(CH₂)_{z}O]ᵥ[CH₂CH₂O]_{w}-PO-(OH)₂

Formula (I); a di-ester corresponding to formula (II) and salts thereof:

{RO[CH₂O]ᵤ[(CH₂)ₓCH(R')(CH₂)_{y}(CH₂)_{z}O]ᵥ[CH₂CH₂O]_{w}}₂PO-(OH)

Formula (II); a tri-ester corresponding to formula (III):

{RO[CH₂O]ᵤ[(CH₂)ₓCH(R')(CH₂)_{y}(CH₂)_{z}O]ᵥ[CH₂CH₂O]_{w}}₃PO Formula (III);

and combinations thereof, wherein:
R is a hydrocarbon radical containing from 6 to 40 carbon atoms;
u, v and w, independently of one another, represent numbers of from 0 to 60;
x, y and z, independently of one another, represent numbers of from 0 to 13;
R' represents hydrogen, alkyl, the sum of x+y+z being ?0. The numbers u, v, and w each represent the degree of alkoxylation. Whereas, on a molecular level, the numbers u, v and w and the total degree of alkoxylation can only be integers, including zero, on a macroscopic level they are mean values in the form of broken numbers.

In formulas (I), (II) and (III), R is linear or branched, acyclic or cyclic, saturated or unsaturated, aliphatic or aromatic, substituted or unsubstituted, preferably a linear or branched, acyclic C₆₋₄₀ alkyl or alkenyl group or a C₁₋₄₀ alkyl phenyl group, more particularly a C₈₋₂₂ alkyl or alkenyl group or a C₄₋₁₈ alkyl phenyl group, more preferably a C₁₂₋₁₈ alkyl group or alkenyl group or a C₆₋₁₆ alkyl phenyl group; u, v, w, independently of one another, is preferably a number from 2 to 20, more preferably a number from 3 to 17 and most preferably a number from 5 to 15;

x, y, z, independently of one another, is preferably a number from 2 to 13, more preferably a number from 1 to 10 and most preferably a number from 0 to 8.

In general, the lower the number of carbon atoms in the R group of the phosphate esters, the more irritating to the skin and the less soluble in water the phosphate ester becomes. In contrast, the higher the number of carbon atoms in the R group, the milder to the skin and the thicker and more waxy the resultant product becomes. Accordingly, for best results, R should have from 12 to 18 carbon atoms. Particularly preferred alkoxylated alkyl phosphate esters for use in the present invention are PPG-5-Ceteth-10 phosphate (CRODAFOS SG(R)), Oleth-3 phosphate (CRODAFOS N3 acid), Oleth-10 phosphate (CRODAFOS N10 acid), and a mixture of Ceteth-10 phosphate and Dicetyl phosphate (CRODAFOS CES) all sold by Croda. Particularly preferred alkyl phosphate esters are Cetyl phosphate (Hostaphat CC 100), Stearyl phosphate (Hostaphat CS 120) from Clariant.

In one embodiment, the alkyl(ether)phosphates are chosen from PPG-5-Ceteth-10 phosphate, Oleth-3 phosphate, Oleth-10 phosphate, Ceteth-10 phosphate, a mixture of Ceteth-10 phosphate and Dicetyl phosphate, Dicetyl phosphate, Cetyl phosphate, Stearyl phosphate, and mixtures thereof.

### Alkylpolyglucosides

The alkyl(poly)glucoside (alkylpolyglycoside) is represented especially by the following general formula:

R₁O-(R₂O)ₜ-(G)ᵥ

wherein:
- R₁ represents a linear or branched alkyl or alkenyl radical comprising 6 to 24 carbon atoms and especially 8 to 18 carbon atoms, or an alkylphenyl radical whose linear or branched alkyl radical comprises 6 to 24 carbon atoms and especially 8 to 18 carbon atoms;
- R₂ represents an alkylene radical comprising 2 to 4 carbon atoms,
- G represents a sugar unit comprising 5 to 6 carbon atoms,
- t denotes a value ranging from 0 to 10 and preferably 0 to 4,
- v denotes a value ranging from 1 to 15 and preferably 1 to 4.

Preferably, the alkylpolyglycoside surfactants are compounds of the formula described above in which:
- R₁ denotes a linear or branched, saturated or unsaturated alkyl radical comprising from 8 to 18 carbon atoms,
- R₂ represents an alkylene radical comprising 2 to 4 carbon atoms,
- t denotes a value ranging from 0 to 3 and preferably equal to 0,
- G denotes glucose, fructose or galactose, preferably glucose;
- the degree of polymerization, i.e. the value of v, possibly ranging from 1 to 15 and preferably from 1 to 4; the mean degree of polymerization more particularly being between 1 and 2.

The glucoside bonds between the sugar units are generally of 1-6 or 1-4 type and preferably of 1-4 type. Preferably, the alkyl(poly)glycoside surfactant is an alkyl(poly)glucoside surfactant. C8/C16 alkyl(poly)glycosides 1,4, and especially decyl glucosides and caprylyl/capryl glucosides, are most particularly preferred.

Among the commercial products, mention may be made of the products sold by the company COGNIS under the names PLANTAREN® (600 CS/U, 1200 and 2000) or PLANTACARE® (818, 1200 and 2000); the products sold by the company SEPPIC under the names ORAMIX CG 110 and ORAMIX NS 10; the products sold by the company BASF under the name LUTENSOL GD 70, or else the products sold by the company CHEM Y under the name AG10 LK.

Preferably, use is made of C8/C16-alkyl(poly)glucosides 1,4, especially as an aqueous 53% solution, such as those sold by Cognis under the reference Plantacare® 818 UP.

In an embodiment, the alkylpolyglucoside is chosen from decyl glucoside, stearyl glucoside, lauryl glucoside, coco-glucoside, cetearyl glucoside, decyl lauryl glucoside, and mixtures thereof.

### Fatty acid alkanolamides

Suitable fatty acid alkanolamides include those formed by reacting an alkanolamine and a C6-C36 fatty acid. Such surfactants can be chosen from mono-alkanolamides and di-alkanolamides of C6-C36 fatty acids, and preferably from mono-alkanolamides and di-alkanolamides of C8-C30 fatty acids or of C8-C24 fatty acids, and may have a C2-3 hydroxyalkyl group. Examples thereof include, but are not limited to:
oleic acid diethanolamide, oleic acid monoisopropanolamide, myristic acid monoethanolamide, soya fatty acids diethanolamide, stearic acid ethanolamide, linoleic acid diethanolamide, behenic acid monoethanolamide, isostearic acid monoisopropanolamide, erucic acid diethanolamide, ricinoleic acid monoethanolamide, coconut isopropanolamide (INCI name: Cocamide MIPA), coconut fatty acid monoethanolamide (INCI name: Cocamide MEA), coconut fatty acid diethanolamide, palm kernel fatty acid diethanolamide, lauric monoethanolamide, lauric diethanolamide, lauric isopropanolamide polyoxyethylene coconut fatty acid monoethanolamide, and mixtures thereof.

In an embodiment, the fatty acid alkanolaminde is chosen from Cocamide MIPA, Cocamide MEA (Coco monoethanolamide), and mixtures thereof.

In one embodiment of the present invention, the at least one nonionic surfactant is selected from cetyl alcohol, stearyl alcohol, cetearyl alcohol (mixture of cetyl alcohol and stearyl alcohol), octyldodecanol, isostearyl alcohol, 2-hexyl decanol, palmityl alcohol, myristyl alcohol, stearyl alcohol, lauryl alcohol, oleic alcohol (or oleyl), linoleyl alcohol (or linoley- ether), linolenic alcohol (or linolenyl) and undecylenic alcohol, and mixtures thereof, and more preferably from cetyl alcohol, stearyl alcohol, and cetearyl alcohol, PPG-5-Ceteth-10 phosphate, Oleth-3 phosphate, Oleth-10 phosphate, Ceteth-10 phosphate, a mixture of Ceteth-10 phosphate and Dicetyl phosphate, Dicetyl phosphate, Cetyl phosphate, Stearyl phosphate, laureth-7, laureth-9, trideceth-10, trideceth-12, C12-13 pareth-3, C12-13 pareth-23, C11-15 pareth-7, PPG-5 ceteth-20, PEG-55 Propylene Glycol Oleate, glycereth-26, decyl glucoside, cetearyl glucoside, decyl lauryl glucoside, stearyl glucoside, coco-glucoside, cocamide MIPA, and mixtures thereof.

The at least one nonionic surfactant is present in the compositions of the present invention in an amount of from about 0.01% to about 10% by weight, preferably from about 0.05% to about 9% by weight, or more preferably from about 0.08% to about 8% by weight, relative to the total weight of the composition, including all ranges and sub ranges therebetween.

In a particular embodiment, the total amount of nonionic surfactants is at about 1%, 1.25%, 1.5%, 1.55%, 1.6%, 1.65%, 1.7%, 1.75%, 1.8%, 1.85%, 1.9%, 1.95%, 2%, 2.05%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 3.0%, 3.25%, 3.5%, 3.75%, 4%, 4.25%, 4.5%, 4.75%, 5%, 5.25%, 5.5%, 5.75%, 6%, 6.25%, 6.5%, 6.75%, 7%, 7.25%, 7.5%, 7.75%, 8%, 8.25%, 8.5 8.75%, 9%, 9.25%, 9.5%, 9.75%, or 10%, by weight, relative to the total weight of the composition.

### CATIONIC CONDITIONING POLYMER

The composition according to the invention comprises one or more cationic polymers. The cationic conditioning polymer used in the invention comprises homopolymers, copolymers, and mixtures thereof.

Non-limiting examples of cationic conditioning polymers useful in the invention include, for example, cationic cellulose derivatives, such as for example polyquaternium-10; cationic gum derivatives such as for example gum derivatives, including particularly guar hydroxypropyltrimonium chloride; polymer derivatives of diallyldimethyl ammonium chloride ("poly-DADMAs") and of methacrylamidopropyltrimethylammonium chloride ("poly-MAPTACs"), and mixtures thereof.

Non-limiting examples of poly-DADMAs and poly- poly-MAPTACs include, polyquaternium-4, polyquaternium-5, polyquaternium-6, polyquaternium-7, polyquaternium-22, polyquaternium-37, polyquaternium-39, polyquaternium-47, polyquaternium-53, and mixtures thereof.

The composition according to the invention preferably comprises cationic polymers known under the INCI names polyquaternium-7, guar hydroxypropyltrimonium chloride, and mixtures thereof.

The cationic polymer is in an amount ranging from about 0.01% to about 4% by weight, preferably from about 0.05% to about 3% by weight, and most preferably from about 0.05% to about 2% by weight, relative to the total weight of the composition.

### AMINO SILICONES

The cosmetic composition according to the invention may further comprise one or more amino silicones. The term "amino silicone" is intended to mean any silicone comprising at least one primary, secondary or tertiary amine or a quaternary ammonium group.

As amino silicone that may be used in the scope of the invention, the following can be cited:
a) polysiloxanes corresponding to formula (A): 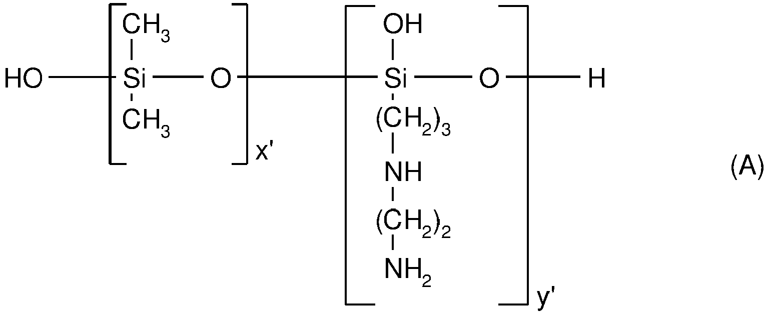 in which x' and y' are integers such that the weight-average molecular weight (Mw) is comprised between about 5000 and 500 000;
b) amino silicones corresponding to formula (B):

   R'aG3-a-Si(OSiG2)n-(OSiGbR'2-b)m-O-SiG3-a-R'a (B)

   in which:
   - G, which may be identical or different, designate a hydrogen atom, or a phenyl, OH or C1-C8 alkyl group, for example methyl, or C1-C8 alkoxy, for example methoxy,
   - a, which may be identical or different, denote the number 0 or an integer from 1 to 3, in particular 0;
   - b denotes 0 or 1, and in particular 1;
   - m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10;
   - R', which may be identical or different, denote a monovalent radical having formula -CqH2qL in which q is a number ranging from 2 to 8 and L is an optionally quaternized amino group chosen from the following groups:
   - NR"-Q-N(R")2
   - N(R")2
   - N+(R")3 A-
   - N+H(R")2 A-
   - N+H2(R") A-
   - N(R")-Q-N+R"H2 A-
   - NR"-Q-N+ (R")2H A-
   - NR"-Q-N+ (R")3 A-,
   in which R", which may be identical or different, denote hydrogen, phenyl, benzyl, or a saturated monovalent hydrocarbon-based radical, for example a C1-C20 alkyl radical; Q denotes a linear or branched CrH2r group, r being an integer ranging from 2 to 6, preferably from 2 to 4; and A- represents a cosmetically acceptable ion, in particular a halide such as fluoride, chloride, bromide or iodide.

A group of amino silicones corresponding to this definition (B) is represented by the silicones called "trimethylsilylamodimethicone" having formula (C): 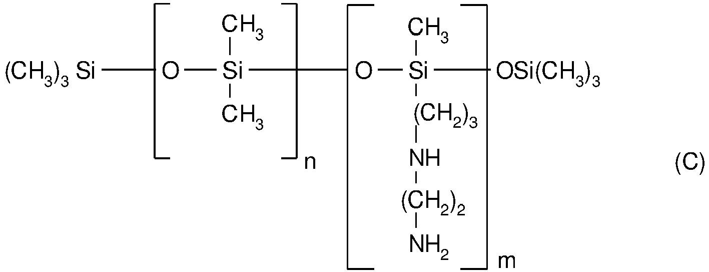 in which n and m have the meanings given above, in formula B.

Another group of amino silicones corresponding to this definition is represented by silicones having the following formulae (D) or (E): 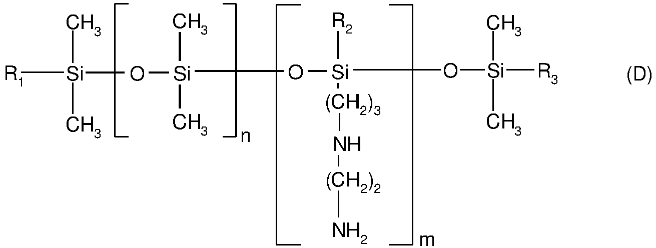 in which:
- m and n are numbers such that the sum (n + m) can range from 1 to 1000, in particular from 50 to 250 and more particularly from 100 to 200, it being possible for n to denote a number from 0 to 999 and in particular from 49 to 249, and more particularly from 125 to 175, and for m to denote a number from 1 to 1000 and in particular from 1 to 10, and more particularly from 1 to 5;
- R1, R2, R3, which may be identical or different, represent a hydroxy or C1-C4 alkoxy radical, where at least one of the radicals R1 to R3 denotes an alkoxy radical.

The alkoxy radical is preferably a methoxy radical.

The hydroxy/alkoxy mole ratio ranges preferably from 0.2:1 to 0.4:1 and preferably from 0.25:1 to 0.35:1 and more particularly equals 0.3:1.

The weight-average molecular weight (Mw) of the silicone ranges preferably from 2000 to 1 000 000, more particularly from 3500 to 200 000. 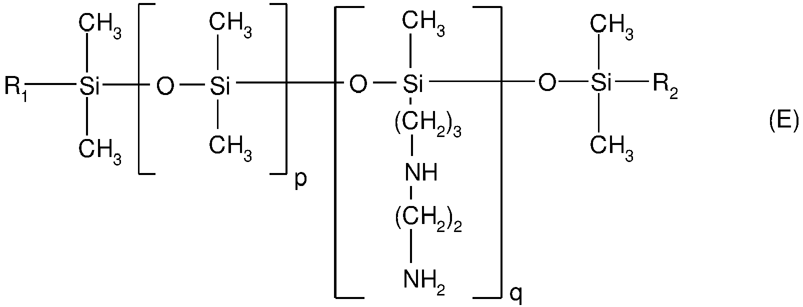 in which:
- p and q are numbers such that the sum (p + q) ranges from 1 to 1000, particularly from 50 to 350, and more particularly from 150 to 250; it being possible for p to denote a number from 0 to 999 and in particular from 49 to 349, and more particularly from 159 to 239 and for q to denote a number from 1 to 1000, in particular from 1 to 10, and more particularly from 1 to 5;
- R1, R2, which are different, represent a hydroxy or C1-C4 alkoxy radical, where at least one of the radicals R1 or R2 denotes an alkoxy radical.

The alkoxy radical is preferably a methoxy radical.

The hydroxy/alkoxy mole ratio ranges generally from 1:0.8 to 1:1.1 and preferably from 1:0.9 to 1:1 and more particularly equals 1:0.95.

The weight-average molecular weight (Mw) of the silicone ranges preferably from 2000 to 200 000, even more particularly 5000 to 100 000 and more particularly from 10 000 to 50 000.

Commercial products corresponding to these silicones having structure (D) or (E) may include in their composition one or more other amino silicones whose structure is different than formulae (D) or (E).

A product containing amino silicones having structure (D) is sold by Wacker under the name Belsil® ADM 652.

A product containing amino silicones having structure (E) is sold by Wacker under the name Fluid WR 1300®.

When these amino silicones are used, one particularly advantageous embodiment consists in using them in the form of an oil-in-water emulsion. The oil-in-water emulsion may comprise one or more surfactants. The surfactants may be of any nature but are preferably cationic and/or nonionic. The number-average size of the silicone particles in the emulsion generally ranges from 3 nm to 500 nanometres. Preferably, in particular as amino silicones having formula (E), microemulsions are used whose average particle size ranges from 5 nm to 60 nanometres (limits included) and more preferably from 10 nm to 50 nanometres (limits included). Accordingly, according to the invention the microemulsions of amino silicone having formula (E) sold as Finish CT 96 E® or SLM 28020® by Wacker can be used.

Another group of amino silicones corresponding to this definition is represented by the following formula (F): 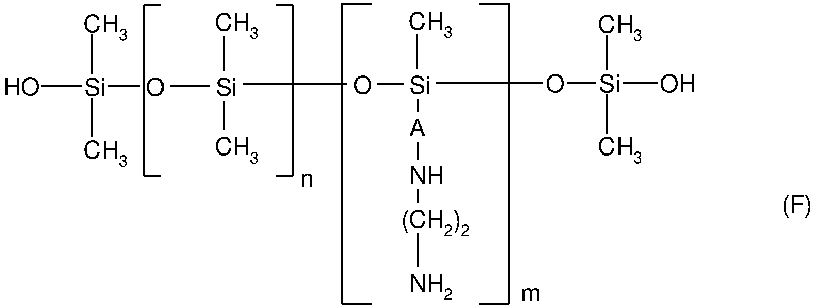 in which:
- m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10;
- A denotes a linear or branched alkylene radical containing from 4 to 8 carbon atoms and preferably 4 carbon atoms. This radical is preferably linear.

The weight-average molecular weight (Mw) of these amino silicones ranges preferably from 2000 to 1 000 000 and even more particularly from 3500 to 200 000.

A preferred silicone of formula (F) is amodimethicone (INCI name) sold under the tradename XIAMETER® MEM-8299 Cationic Emulsion by Dow Corning.

Another group of amino silicones corresponding to this definition is represented by the following formula (G): 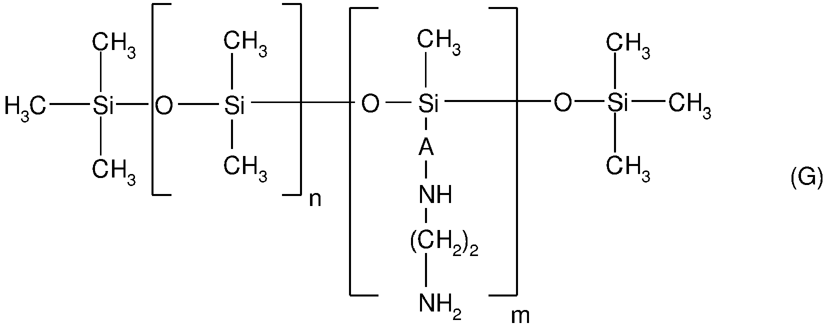 in which:
- m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10;
- A denotes a linear or branched alkylene radical containing from 4 to 8 carbon atoms and preferably 4 carbon atoms. This radical is preferably branched.

The weight-average molecular weight (Mw) of these amino silicones ranges preferably from 500 to 1 000 000 and even more particularly from 1000 to 200 000.

A silicone having this formula is for example DC2-8566 Amino Fluid by Dow Corning.
c) amino silicones corresponding to formula (H): 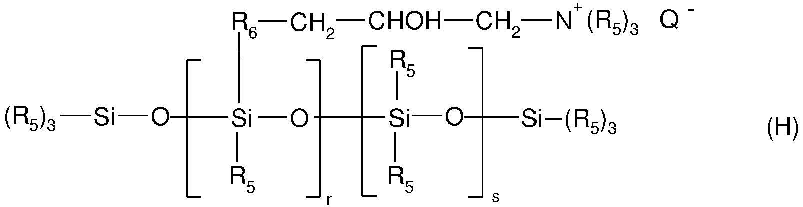 in which:
- R5 represents a monovalent hydrocarbon-based radical containing from 1 to 18 carbon atoms, and in particular a C1-C18 alkyl or C2-C18 alkenyl radical, for example methyl;
- R6 represents a divalent hydrocarbon-based radical, in particular a C1-C18 alkylene radical or a divalent C1-C18, for example C1-C8, alkylenoxy radical linked to the Si via an SiC bond;
- Q- is an anion such as a halide ion, in particular chloride, or an organic acid salt (for example acetate);
- r represents a mean statistical value from 2 to 20 and in particular from 2 to 8;
- s represents a mean statistical value from 20 to 200 and in particular from 20 to 50.

Such amino silicones are described more particularly in patent US 4 185 087.
d) quaternary ammonium silicones having formula (I): 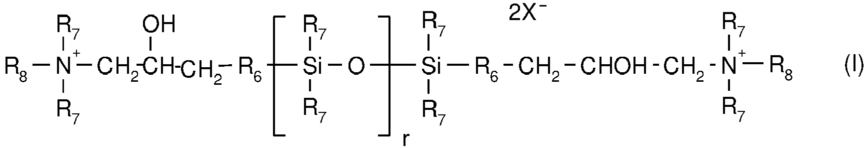 in which:
- R7, which may be identical or different, represent a monovalent hydrocarbon-based radical containing from 1 to 18 carbon atoms, and in particular a C1-C18 alkyl radical, a C2-C18 alkenyl radical or a ring containing 5 or 6 carbon atoms, for example methyl;
- R6 represents a divalent hydrocarbon-based radical, in particular a C1-C18 alkylene radical or a divalent C1-C18, for example C1-C8, alkylenoxy radical linked to the Si via an SiC bond;
- R8, which may be identical or different, represent a hydrogen atom, a monovalent hydrocarbon-based radical containing from 1 to 18 carbon atoms, and in particular a C1-C18 alkyl radical, a C2-C18 alkenyl radical or a -R6-NHCOR7 radical;
- X- is an anion such as a halide ion, in particular chloride, or an organic acid salt (for example acetate);
- r represents a mean statistical value from 2 to 200 and in particular from 5 to 100;

These silicones are described, for example, in patent application EP-A 0 530 974.
e) amino silicones having formula (J): 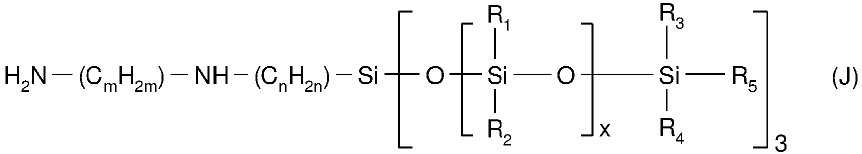 in which:
   - R1, R2, R3 and R4, which may be identical or different, denote a C1 - C4 alkyl radical or a phenyl group;
   - R5 denotes a C1-C4 alkyl radical or a hydroxyl group;
   - n is an integer ranging from 1 to 5;
   - m is an integer ranging from 1 to 5;
   and in which x is chosen such that the amine number is between 0.01 and 1 meq/g;
f) multiblock polyoxyalkylenated amino silicones, of type (AB)n, A being a polysiloxane block and B being a polyoxyalkylenated block containing at least one amine group.

Said silicones are preferably constituted of repeating units having the following general formulae:

[-(SiMe2O)xSiMe2 - R -N(R")- R'-O(C2H4O)a(C3H6O)b-R'-N(H)-R-]

or alternatively

[-(SiMe2O)xSiMe2 - R -N(R")- R' - O(C2H4O)a(C3H6O)b-]

in which:
- a is an integer greater than or equal to 1, preferably ranging from 5 to 200, more particularly ranging from 10 to 100;
- b is an integer comprised between 0 and 200, preferably ranging from 4 to 100, more particularly between from 5 and 30;
- x is an integer ranging from 1 to 10 000, more particularly from 10 to 5000;
- R" is a hydrogen atom or a methyl;
- R, which may be identical or different, represent a divalent linear or branched C2-C12 hydrocarbon-based radical, optionally including one or more heteroatoms such as oxygen; preferably, R denotes an ethylene radical, a linear or branched propylene radical, a linear or branched butylene radical, or a - CH2CH2CH2OCH(OH)CH2- radical; preferentially R denotes a-CH2CH2CH2OCH(OH)CH2- radical;
- R', which may be identical or different, represent a divalent linear or branched C2-C12 hydrocarbon-based radical, optionally including one or more heteroatoms such as oxygen; preferably, R' denotes an ethylene radical, a linear or branched propylene radical, a linear or branched butylene radical, or a-CH2CH2CH2OCH(OH)CH2- radical; preferentially R' denotes -CH(CH3)-CH2-.

The siloxane blocks preferably represent between 50 and 95 mol% of the total weight of the silicone, more particularly from 70 to 85 mol%.

The amine content is preferably between 0.02 and 0.5 meq/g of copolymer in a 30% solution in dipropylene glycol, more particularly between 0,05 and 0,2.

The weight-average molecular weight (Mw) of the silicone is preferably comprised between 5000 and 1 000 000, more particularly between 10 000 and 200 000.

Mention may be made especially of the silicones sold under the names Silsoft™ A-843 or Silsoft™ A+ by Momentive.
g) the alkylamino silicones corresponding to formula (K) below: 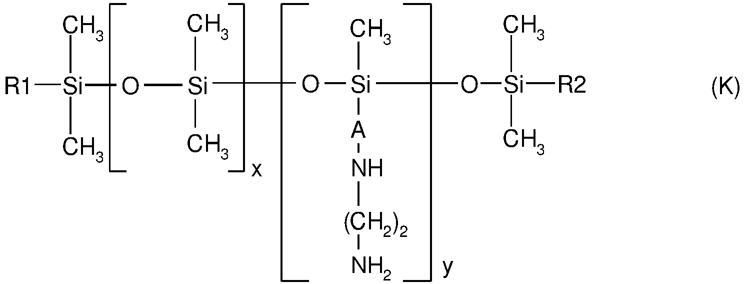 in which:
- x and y are numbers ranging from 1 to 5000; preferably, x ranges from 10 to 2000 and especially from 100 to 1000; preferably, y ranges from 1 to 100;
- R1 and R2, which may be identical or different, preferably identical, are linear or branched, saturated or unsaturated alkyl radicals, comprising 6 to 30 carbon atoms, preferably 8 to 24 carbon atoms and especially 12 to 20 carbon atoms;
- A denotes a linear or branched alkylene radical containing from 2 to 8 carbon atoms,

Preferably, A comprises 3 to 6 carbon atoms, especially 4 carbon atoms; preferably, A is branched. Mention may be made especially of the following divalent radicals: -CH2CH2CH2 and -CH2CH(CH3)CH2-.

Preferably, R1 and R2, which may be identical or different, are saturated linear alkyl radicals comprising 6 to 30 carbon atoms, preferably 8 to 24 carbon atoms and especially 12 to 20 carbon atoms; mention may be made in particular of dodecyl, tetradecyl, pentadecyl, hexadecyl, heptadecyl, octadecyl, nonadecyl and eicosyl radicals; and preferentially, R1 and R2, which may be identical or different, are chosen from hexadecyl (cetyl) and octadecyl (stearyl) radicals.

Preferentially, the silicone is of formula (K) with:
- x ranging from 10 to 2000 and especially from 100 to 1000;
- y ranging from 1 to 100;
- A comprising 3 to 6 carbon atoms and especially 4 carbon atoms; preferably, A is branched; and more particularly A is chosen from the following divalent radicals: -CH2CH2CH2 and -CH2CH(CH3)CH2-; and
- R1 and R2, which may be identical or different, being linear, saturated alkyl radicals comprising 6 to 30 carbon atoms, preferably 8 to 24 carbon atoms and especially 12 to 20 carbon atoms; chosen in particular from dodecyl, tetradecyl, pentadecyl, hexadecyl, heptadecyl, octadecyl, nonadecyl and eicosyl radicals; preferentially, R1 and R2, which may be identical or different, being chosen from hexadecyl (cetyl) and octadecyl (stearyl) radicals.

A preferred silicone of formula (K) is bis-cetearyl amodimethicone (INCI name).

Mention may be made especially of the silicone sold under the name Silsoft™ AX by Momentive.

In one embodiment, the amino silicones according to the invention are chosen from amodimethicone, trideceth-9 PG amodimethicone, PEG-40/PPG-8 Methylaminopropyl/hydroxypropyl dimethicone copolymer, and mixtures thereof.

Preferably, the amino silicones according to the invention are chosen from the amino silicones of formula (F). A preferred silicone of formula (F) is amodimethicone (INCI name) sold under the tradename XIAMETER® MEM-8299 Cationic Emulsion by Dow Corning.

When present in the composition according to the invention, the amino silicone is in an amount ranging from about 0.01% to about 5% by weight, preferably from about 0.05% to about 3% by weight, and most preferably from about 0.1% to about 2% by weight, relative to the total weight of the composition, including all ranges and sub ranges therebetween.

### NEUTRALIZING AGENTS

The compositions of the present invention may further comprise at least one neutralizing agent wherein the at least one neutralizing agent is selected from organic amines, alkali metal hydroxides, alkali earth metal hydroxides, alkali metal carbonates, alkali metal phosphates, and mixtures thereof,

Organic amines may be chosen from organic amines comprising one or two primary, secondary, or tertiary amine functions, and at least one linear or branched C1-C8 alkyl groups bearing at least one hydroxyl radical.

Organic amines may also be selected cyclic amines and other cyclic compounds, saturated or unsaturated, having one or more nitrogen atoms within the ring, and mixtures thereof.

The organic amines may be chosen from the ones having a pKb at 25°C of less than 12, such as less than 10 or such as less than 6. It should be noted that this is the pKb corresponding to the function of highest basicity.

Organic amines may also be chosen from alkanolamines such as mono-, di- or trialkanolamines, comprising one to three identical or different C1-C4 hydroxyalkyl radicals, ethylamines, ethyleneamines, quinoline, aniline and cyclic amines, such as pyrroline, pyrrole, pyrrolidine, imidazole, imidazolidine, imidazolidinine, morpholine, pyridine, piperidine, pyrimidine, piperazine, triazine and derivatives thereof.

Among the compounds of the alkanolamine type that may be mentioned include but not limited to: monoethanolamine (also known as monoethanolamine or MEA), diethanolamine, triethanolamine, monoisopropanolamine, diisopropanolamine, N-dimethylaminoethanolamine, 2-amino-2-methyl-1-propanol, triisopropanolamine, 2-amino-2-methyl-1,3-propanediol, 3-amino-1,2-propanediol, 3-dimethylamino-1,2-propanediol, 2-amino-2-methyl-1-propanol (aminomethyl propanol), and tris(hydroxymethylamino)methane.

Other examples include but are not limited to: 1,3-diaminopropane, 1,3-diamino-2-propanol, spermine, and spermidine.

In some embodiments, the organic amines are chosen from amino acids.

As non-limiting examples, the amino acids that may be used may be of natural or synthetic origin, in L, D, or racemic form, and comprise at least one acid function chosen from, for instance, carboxylic acid, sulfonic acid, phosphonic acid, and phosphoric acid functions. The amino acids may be in their neutral or ionic form.

Further as non-limiting examples, the amino acids may be chosen from basic amino acids comprising an additional amine function optionally included in a ring or in a ureido function.

Amino acids that may be used in the present disclosure include but are not limited to: aspartic acid, glutamic acid, alanine, arginine, ornithine, citrulline, asparagine, carnitine, cysteine, glutamine, glycine, histidine, lysine, isoleucine, leucine, methionine, N-phenylalanine, proline, serine, taurine, threonine, tryptophan, tyrosine, ornithine, citrulline, and valine.

In some embodiments, the organic amines are chosen from organic amines of heterocyclic type. Besides histidine that has already been mentioned in the amino acids, non-limiting mention may also be made of pyridine, piperidine, imidazole, 1,2,4-triazole, tetrazole, and benzimidazole.

In some embodiments, the organic amines are chosen from amino acid dipeptides. Amino acid dipeptides that may be used in the present disclosure include but not limited to: carnosine, anserine, and baleine.

In some embodiments, the organic amines are chosen from compounds comprising a guanidine function. Organic amines of this type that may be used in the present disclosure include, besides arginine that has already been mentioned as an amino acid, creatine, creatinine, 1,1-dimethylguanidine, 1,1-diethylguanidine, glycocyamine, metformin, agmatine, N-amidinoalanine, 3-guanidinopropionic acid, 4-guanidinobutyric acid, and 2-([amino(imino)methyl]amino)ethane-1 -sulfonic acid.

The alkali metal phosphates and carbonates that may be used are, for example, sodium phosphate, potassium phosphate, sodium carbonate, sodium bicarbonate, potassium carbonate, potassium bicarbonate, and their derivatives.

Neutralizing agents may also be chosen from alkali metal hydroxides, alkaline-earth metal hydroxides, transition metal hydroxides, quaternary ammonium hydroxides, organic hydroxides, and mixtures thereof. Suitable examples are ammonium hydroxide, sodium hydroxide, potassium hydroxide, lithium hydroxide, rubidium hydroxide, caesium hydroxide, francium hydroxide, beryllium hydroxide, magnesium hydroxide, calcium hydroxide, strontium hydroxide, barium hydroxide, molybdenum hydroxide, manganese hydroxide, zinc hydroxide, cobalt hydroxide, cadmium hydroxide, cerium hydroxide, lanthanum hydroxide, actinium hydroxide, thorium hydroxide, aluminium hydroxide, guanidinium hydroxide and mixtures thereof.

According to at least one embodiment, the at least one neutralizing agent is chosen from aminomethyl propanol, sodium hydroxide, potassium hydroxide, lithium hydroxide, aminomethyl propanediol, triisopropanol amine, dimethylstearylamine, dimethyl/tallowamine, lysine, ornithine, arginine, monoethanolamine, triethanolamine, calcium hydroxide, calcium bicarbonate, and mixtures thereof.

According to another preferred embodiment, the at least one neutralizing agent is chosen from aminomethyl propanol, sodium hydroxide, lithium hydroxide, calcium hydroxide, monoethanolamine, and mixtures thereof.

The at least neutralizing agent may be present in an amount of from 0.01% to 3% by weight, preferably from 0.02% to 2.75% by weight, more preferably from 0.025% to 2.5% by weight, even more preferably from 0.03% to 2% by weight, relative to the total weight of the composition, including all ranges and sub ranges therebetween.

When the at least neutralizing agent is selected from aminomethyl propanol, it is present in an amount of from 0.1% to 6.3% by weight, preferably from 0.2% to 5.5% by weight, more preferably from 0.3% to 5% by weight, even more preferably from 0.3% to 4.6% by weight, relative to the total weight of the composition, including all ranges and sub ranges therebetween.

When the at least neutralizing agent is selected from sodium hydroxide, it is present in an amount of from 0.1% to 4.1% by weight, preferably from 0.15% to 3.5% by weight, more preferably from 0.2% to 3% by weight, even more preferably from 1% to 3% by weight, relative to the total weight of the composition, including all ranges and sub ranges therebetween.

When the at least neutralizing agent is selected from monoethanolamine, it is present in an amount of from 0.1% to 4.1% by weight, preferably from 0.15% to 3.5% by weight, more preferably from 0.2% to 3% by weight, even more preferably from 1% to 3% by weight, relative to the total weight of the composition, including all ranges and sub ranges therebetween.

### pH

The pH of the compositions of the present invention may range from about 2 to less than 7, or from about pH 2.5 to about 6.5, or from about pH 3 to about 6, or from about pH 3 to about 5.2, such as from about pH 3 to about 5, or preferably from about pH 3 to about 4.8, or more preferably from about pH 3 to about 4.5, or even more preferably from about pH 3 to about 4, including all ranges and sub ranges therebetween.

In some embodiments, the pH of the compositions of the present invention can be at about 2, 2.5, 2.75, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5.

The desired pH may be obtained by using one or more of the neutralizing agents described above.

### WATER

The composition according to the invention comprises water. The water used may be sterile demineralized water and/or a floral water such as rose water, cornflower water, chamomile water or lime water, and/or a natural thermal or mineral water such as, for example: water from Vittel, water from the Vichy basin, water from Uriage, water from La Roche Posay, water from La Bourboule, water from Enghienles-Bains, water from Saint Gervais-les-Bains, water from Neris-les-Bains, water from Allevar-les-Bains, water from Digne, water from Maizieres, water from Neyracles-Bains, water from Lons-le-Saunier, water from Eaux Bonnes, water from Rochefort, water from Saint Christau, water from Les Fumades, water from Tercisles-Bains or water from Avene. Water may also comprise reconstituted thermal water, that is to say a water comprising trace elements such as zinc, copper, magnesium, etc., reconstituting the characteristics of a thermal water.

The composition according to the invention comprises water in an amount ranging from about 50% to about 99% by weight, preferably from about 65% to about 95% by weight, and most preferably from about 70% to about 92% by weight, relative to the total weight of the composition.

### ORGANIC SOLVENT (OPTIONAL)

The compositions according to the invention optionally may comprise one or more organic solvent.

Non-limiting examples of organic solvents useful in the invention include, for example, glycols and polyols containing from 2 to 8 carbon atoms, such as ethylene glycol, propylene glycol, 1,3-butylene glycol, pentylene glycol, hexylene glycol, dipropylene glycol, glycerin, and mixtures thereof.

Other non-limiting examples of organic solvents useful in the invention include, for example, lower carbon alcohols such as ethanol, propanol, isopropanol, and mixtures thereof.

When present in the composition according to the invention, the watersoluble solvent is preferably present in the composition according to the invention in an amount of from about 0.01% to 10% by weight, preferably in an amount of from about 0.05% to 5% by weight, and most preferably 0.1% to 1% by weight, based on the total weight of the composition.

The composition according to the invention may also comprise at least one additional component selected from organic solvents, silicones other than amino functional silicones, plasticizers, opacifiers, plant/vegetable oils, hydrocarbons, lower alkanes, fragrance, preservatives, pH adjusters, plant extracts, salts, vitamins, sunscreens, colorants, and mixtures thereof.

The silicones other than amino functional silicones can be chosen from dimethicone and dimethicone copolyol compounds such as oxypropylenated and/or oxyethylenated polydimethyl(methyl)siloxane, oxypropylenated and/or oxyethylenated polymethyl (C8-C22) alkyl dimethyl methyl siloxane, and mixtures thereof, and wherein the dimethicone copolyol compounds are preferably selected from Dimethicone PEG-8 Benzoate, Dimethicone PEG-7 Phosphate, Dimethicone PEG-8 Phosphate, Dimethicone PEG-10 Phosphate, PEG-7 Dimethicone, PEG-8 Dimethicone, PEG-9 Dimethicone, PEG-10 Dimethicone, PEG-12 Dimethicone, PEG-14 Dimethicone, PEG-17 Dimethicone, PEG/PPG-3/10 Dimethicone, PEG/PPG-4/12 Dimethicone, PEG/PPG-17/18 Dimethicone, cetyl PEG/PPG- 10/1 dimethicone, and mixtures thereof.

A person skilled in the art will take care to select the optional additional components and the amount thereof such that they do not harm the properties of the compositions of the present invention.

These additional components are generally present in the composition according to the invention in an amount ranging from about 0 to about 20% by weight relative to the total weight of the composition.

In one embodiment, the hair cosmetic composition of the present invention is an aqueous composition and may be provided in the form of a shampoo or cleanser.

In one embodiment, the present invention is related to a method or process of cleansing hair, wherein the hair cosmetic composition is applied onto hair (wet or dry), massaged into the hair fibers, then rinsed out with water.

In one embodiment, the present invention is related to a process or method of cleansing hair according to the general protocol or treatment cycle involving the steps of:
a) optionally, washing/rinsing the hair with a shampoo having a neutral pH and/or rinsing the hair with water, followed by allowing the hair to air dry or by drying the at a temperature ranging from room temperature up to about 100ºC by using for example, a blow dryer, while optionally applying a smoothing action on the hair;
b) applying the composition onto the hair;
c) allowing the composition to remain on the hair for a period of time ranging from about 1 to about 10 minutes or from about 1 to about 5 minutes;
d) rinsing the hair with water;
e) drying the hair at a temperature ranging from room temperature up to about 40ºC, while optionally applying a smoothing action on the hair;
f) passing a flat iron over the hair swatch at least once, or such as two times up to 10 times or such as three times up to 10 times wherein the flat iron is employed at a temperature of about 100 ºC, or ranging from about 100°C to about 250°C or from about 110°C to about 230°C or from about 110°C to about 210°C or from about 120°C to about 200°C or from about 150°C to about 190°C, or from about 190°C to about 230°C, including ranges and sub-ranges therebetween, or at a temperature of about 230°C or about 225°C or about 220°C or about 210°C or about 200°C or about 190°C or about 180°C or about 150°C or about 100°C and preferably at about 230°C; and
g) washing/rinsing the hair with a shampoo having a neutral pH and/or rinsing the hair with water, followed by allowing the hair to air dry, while optionally applying a smoothing action on the hair.

The smoothing action may be conducted by brushing or combing or passing the fingers through the hair.

Drying the hair at a temperature ranging from room temperature up to about 100ºC may be accomplished by drying the hair with a blow dryer device or using other heat sources such as a flat iron, a hair dryer, a heat lamp, a heat wand, or other similar devices.

In various embodiments, the flat iron is passed over the hair at least once or from one time up to 10 times or from 2 times up to 10 times or from 3 times up to 10 times or from 5 times up to 10 times or from 6 times up to 10 times or 10 times.

The composition can be applied onto the hair using an applicator device or with the hands or gloved hands or with the fingers. A suitable applicator device is an applicator brush or applicator comb or applicator spatula or a dispenser or applicator tip attached to the container holding the composition.

The method or treatment cycle described above may be repeated over a period of days or weeks.

The cosmetic effects imparted by the compositions and accompanying methods of treating the hair of the present invention may be evaluated by visually assessing the appearance of the hair after processing the hair according to the methods of the invention. Another type of evaluation can also involve sensorial evaluations of the hair.

It was surprisingly and unexpectedly discovered that the hair contacted with the compositions of the invention and treated according to the methods of the invention visually generally appeared to be more extended and/or straight, less volumized, and less frizzy compared to hair contacted with compositions that did not contain thiolactic acid. It was also surprisingly and unexpectedly discovered that the hair contacted with the compositions of the invention and treated according to the methods of the invention were smoother to the touch, more manageable, more disciplined (i.e., less or no fly-aways), and exhibited more regularity with respect to shape and appearance. These effects were even more observable after subjecting the hair to multiple treatment or application cycles.

The compositions of the present invention may be packaged in any suitable container such as a tube, a jar or a bottle. In certain embodiments, the composition can be packaged in a tube or bottle, for example, a squeeze tube or squeeze bottle. Additionally, an applicator device can be attached or connected to the opening of the packaging/squeeze tube or bottle wherein the applicator device is a brush or a comb with teeth such that the ends of the teeth have openings from which the composition of the invention can flow through and be applied directly onto the hair.

The composition of the present invention may also be provided as component of a kit for treating or conditioning hair wherein the kit can additionally contain other components such as a shampoo.

While the invention has been described with reference to a preferred embodiment, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims.

The compositions according to the invention can be manufactured by known processes used generally in the cosmetics, including the processes described in the examples below.

The following Examples are within the scope of the disclosed embodiments.

The ingredient amounts in the compositions/formulas described below are expressed in % by weight, based on the total weight of the composition.

### EXAMPLES

### Example A - Formulations

Shampoo formulas of the present invention can be prepared in water according to Table 1.

| **Ingredient** | **% by Weight (active matter, "AM")** |
|---|---|
| Cationic surfactants (behentrimonium chloride, cetrimonium chloride, behentrimonium methosulfate) | 0.1-10 |
| Amphoteric surfactants (eg, coco betaine, cocamidopropylbetaine, cocoamphoacetate, cocoamphodiacetate, and their salts, or mixtures thereof) | 0.1-10 |
| Thiolactic acid | 0.5-15 |
| Thickening agents (e.g., carbomer, cellulose compounds, starch derivatives) | 0.01-2 |
| Nonionic surfactants (e.g., fatty alcohols, alkoxylated fatty alcohols, alkyl(ether)phosphates, alkylpolyglucosides, fatty acid alkanolamides, or mixtures thereof) | 0.01-10 |
| Cationic conditioning polymers compounds (e.g., quaternary ammonium compounds such as polyquaternium compounds) | 0.01-4 |
| Amino Silicones (eg, amodimethicone) | 0.1-10 - |
| Water, organic solvent, and additional components such as silicones other than amino silicones, fragrance, preservatives, pH adjusting agents, plant extracts, plant oils, hydrolyzed proteins vitamins, salt, and fragrances, opacifiers (e.g., glycol distearate), plasticizers, salts, vitamins, sunscreens, lower alkanes, hydrocarbons, colorants, or mixtures thereof (as needed or desired) | Q.S. 100 |

Examples of the inventive shampoo formulas containing thiolactic acid and comparative formulas which do not contain thiolactic acid were prepared and are presented in the table below. These formulas are mild foaming compositions and are substantially free of anionic surfactants.

**Table 2. Shampoo Formulas**

| | **Comparative formula** | **Inventive formulas** | | | |
|---|---|---|---|---|---|
| | **Example 1** | **Example 2** | **Example 3** | **Example 4** | **Example 5** |
| US INCI NAME | % by weight | | | | |
| COCO-BETAINE | 7 (2.1% AM) | 7 (2.1% AM) | 7 (2.1% AM) | 7 (2.1% AM) | 7 (2.1% AM) |
| BEHENTRIMONIUM CHLORIDE | 3 (2.37% AM) | 3 (2.37% AM) | 3 (2.37% AM) | 3 (2.37% AM) | 3 (2.37% AM) |
| THIOLACTIC ACID | - | 1 | 2.5 | 5 | 8 |
| CETYL HYDROXYETHYLCEL LULOSE (NATROSOL™ Plus 330 CS from Ashland) | 0.23 | 0.23 | 0.23 | 0.23 | 0.23 |
| CETEARYL ALCOHOL | 7 | 7 | 7 | 7 | 7 |
| POTATO STARCH MODIFIED (STRUCTURE® SOLANACE from AkzoNobel) | 1.2 (1.0% AM) | 1.2 (1.0% AM) | 1.2 (1.0% AM) | 1.2 (1.0% AM) | 1.2 (1.0% AM) |
| GUAR HYDROXYPROPYL TR IMONIUM CHLORIDE | 0.12 | 0.12 | 0.12 | 0.12 | 0.12 |
| POLYQUATERNIUM-7 | 0.4 (0.036% AM) | 0.4 (0.036% AM) | 0.4 (0.036% AM) | 0.4 (0.036% AM) | 0.4 (0.036% AM) |
| AMODIMETHICONE (and) TRIDECETH-6 (and) CETRIMONIUM CHLORIDE (XIAMETER® MEM-8299 Cationic Emulsion from Dow Corning; 57.5% AM) | 1 | 1 | 1 | 1 | 1 |
| LIMNANTHES ALBA (MEADOWFOAM) SEED OIL | 0.01 | 0.01 | 0.01 | 0.01 | 0.01 |
| Additional Components and water | QS 100 | QS 100 | QS 100 | QS 100 | QS 100 |
| pH | 5.0 | 3.5 | 3.5 | 3.5 | 3.5 |

In making the Formulas in Table 2, the following procedure was used:

The cationic surfactant (behentrimonium chloride) was combined with cetyl hydroxyethyl cellulose, guar hydroxypropyltrimonium chloride, fatty alcohol (cetearyl alcohol) with water (20% by weight of the total amount of water in the formula) with stirring to hydration and heating up to 70°C for 10 minutes. The amphoteric surfactant (coco-betaine) was added and the solution was mixed well for 15 minutes. As a separate mixture, potato starch modified and meadow foam oil were added to water (in an amount of about 15% by weight of the total amount of water in the formula). The separate mixture was added to the rest of the formula and the resulting mixture was mixed well and cooled to room temperature. The amino silicone (amodimethicone emulsion, polyquaternium-7, thiolactic acid, and remainder of the water were added. If necessary, pH was adjusted with Sodium Hydroxide.

Optional ingredients and additives were included in the formulas: for example, organic solvents, fragrance, preservatives, plant oils and extracts, neutralizing agents, cationic conditioning polymers, silicones, opacifiers, plasticizers, salts, vitamins, sunscreens, lower alkanes, hydrocarbons, colorants, or mixtures thereof.

### Example B: Performance Evaluation

### Application Protocol

The formulas above were used to treat hair swatches according to the following hair treatment protocol:
1. The hair swatches were washed with a shampoo having a neutral pH, rinsed with water, and the hair was dried at a temperature ranging from room temperature up to about 100ºC by using for example, a blow dryer, while optionally applying a smoothing action on the hair (this step may be an optional step).
2. Each formula was spread along the length of the hair swatches and allowed to remain on the hair for about 5 minutes.
3. The hair swatches were rinsed with water and the hair was dried at a temperature ranging from room temperature up to about 100ºC by using for example, a blow dryer, while optionally applying a smoothing action on the hair.
4. A flat iron was passed over each hair swatch 3 times at 230ºC.
5. The hair swatches were washed with a shampoo having a neutral pH, rinsed with water, and allowed to air dry.
6. The hair swatches were subjected to sensorial and visual assessments with respect to the attributes of straightening, volume and frizz based on a standardized rating scale of 1 to 4, with 4 being the best rating (highest degree of straightening, least amount of volume and least amount of frizziness).

In order to evaluate the long-lasting performance of the formulas on hair over time, the hair swatches were also subjected to multiple applications (from 2 to 5 applications) according to the protocol above based on the common practice of people of washing/cleansing their hair 2 to 3 times a week. One application corresponds to one treatment cycle comprising steps 1 to 5. Five applications or five treatment cycles were designed to simulate washing/cleansing of hair over a two week period (people tend to wash their hair 2 to 3 times a week).

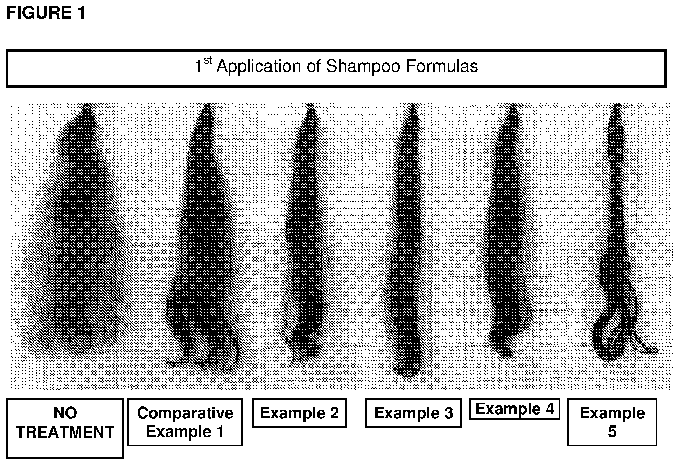

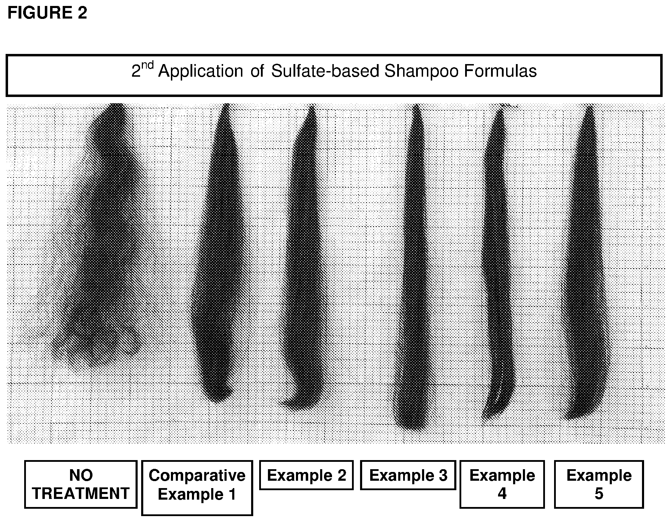

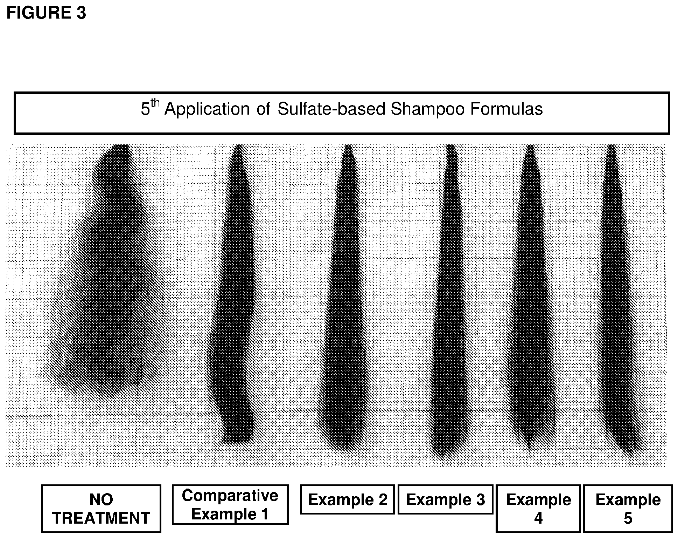

Figures 1 to 3 are photographic images of the hair swatches treated with the inventive and comparative shampoo formulas and subjected to several applications or treatment cycles (at 1st, 2nd, and 5th applications).

### Performance results

### A. First Application

| **Attributes** | **No treatment (water only)** | **Comparative Example 1** | **Example 2** | **Example 3** | **Example 4** | **Example 5** |
|---|---|---|---|---|---|---|
| Straight | 1.0 | 2.0 | 2.5 | 2.5 | 2.5 | 2.0 |
| Volume | 1.0 | 2.0 | 3.0 | 3.0 | 3.0 | 4.0 |
| Frizz | 1.0 | 2.0 | 3.0 | 3.5 | 3.0 | 3.5 |

### SENSORIAL & VISUAL EVALUATIONS

Manageability
Volume Reduction
Frizz Control
Smooth Touch
Cosmeticity
Discipline
Natural Touch

### B. Second Application

| **Attributes** | **No treatment (water only)** | **Comparative Example 1** | **Example 2** | **Example 3** | **Example 4** | **Example 5** |
|---|---|---|---|---|---|---|
| Straightening | 1.0 | 3.0 | 2.5 | 3.0 | 2.5 | 2.5 |
| Volume | 1.0 | 2.5 | 3.0 | 3.5 | 3.5 | 3.5 |
| Frizz | 1.0 | 3.5 | 3.0 | 3.0 | 3.0 | 3.5 |

### SENSORIAL & VISUAL EVALUATIONS

Straightening
Volume Reduction
Frizz Control
Smooth Touch
Cosmeticity
Regularity
Discipline
Natural Touch

### C. Fifth Application

| **Attributes** | **No treatment (water only)** | **Comparative Example 1** | **Example 2** | **Example 3** | **Example 4** | **Example 5** |
|---|---|---|---|---|---|---|
| Straightening | 1.0 | 3.5 | 3.5 | 3.5 | 4.0 | 3.5 |
| Volume | 1.0 | 3.5 | 3.5 | 3.5 | 3.5 | 3.5 |
| Frizz | 1.0 | 3.5 | 3.5 | 3.5 | 3.0 | 3.0 |

### SENSORIAL & VISUAL EVALUATIONS

Straightening
Volume Reduction
Frizz Control
Smooth Touch
Cosmeticity
Regularity
Discipline
Natural Touch

### Summary of results:

After the 1st application, the ratings for the hair swatches treated with the inventive formulas at 1, 2.5, 5, and 8% by weight of thiolactic acid were higher to the ratings for the hair treated with the comparative formulas with respect to straightening, volume and frizz. After the 2nd application, the ratings for the hair swatches treated with the inventive formulas at 1, 2,5, 5, and 8% by weight of thiolactic acid were higher to the ratings for the hair treated with the comparative formulas with respect to volume. After the 5th application, the ratings for the hair swatches treated with the inventive formulas at 1, 2.5, 5, and 8% by weight of thiolactic acid were comparable or higher than the ratings for the hair treated with the comparative formulas with respect to straightening and volume. After the 5th application, the ratings for the hair swatches treated with the inventive formulas at 2.5, 5, and 8% by weight of thiolactic acid were comparable or higher than the ratings for the hair treated with the comparative formulas with respect to straightening. While the rest of the results showed lower ratings for the hair treated with the inventive shampoos with respect to some of the attributes at various application cycles and at certain levels of thiolactic acid, the hair treated with the inventive sulfate-based shampoos provided better sensorial (by feel) attributes of cosmeticity, smooth touch and natural touch as compared to the hair treated with the comparative formula that did not contain thiolactic acid.

Also, the effects of multiple applications were observed wherein the ratings and the visual appearance of the hair swatches treated with the inventive formulas at 1, 2.5, 5, and 8% by weight of thiolactic acid generally improved or remained comparable, over the five applications. The most dramatic differences in attribute ratings between the treatments with the inventive formulas and the comparative formulas and also among the three application stages were observed after the 1^{st} application. These results are also evident in Figures 1 to 3.

Overall, the formulas of the invention imparted hair care and manageability properties to the hair by providing the cosmetic effects of straightening, volume control or reduction, frizz control or less frizziness, cosmetic feel, smooth feel, discipline, regularity and natural touch.

## Claims

1. A hair cosmetic composition comprising:
(a) at least one cationic surfactant;
(b) at least one amphoteric surfactant;
(c) at least one thiol-based compound selected from thiolactic acid, their salts, and mixtures thereof;
(d) at least one thickening agent;
(e) at least one nonionic surfactant;
(f) at least one cationic conditioning polymer;
(g) optionally, at least one amino silicone; and
(h) water; and
wherein the pH of the composition ranges from 2 to less than 7.

2. The hair cosmetic composition according to the preceding claim, wherein the at least one cationic surfactant is selected from optionally polyoxyalkenylated primary, secondary, tertiary fatty amine salts, quaternary ammonium salts, and mixtures thereof, and preferably from quaternary ammonium salts, and more preferably from cetrimonium chloride, behentrimonium chloride, behentrimonium methosulfate, dipalmitoylethylhydroxyethylmethylammonium methosulfate, and mixtures thereof.

3. The hair cosmetic composition according to any one of the preceding claims, wherein the at least one cationic surfactant is present in an amount of from about 0.1% to about 10% by weight, preferably from about 0.5% to about 8% by weight, and most preferably from about 0.7% to about 5% by weight, relative to the total weight of the composition.

4. The hair cosmetic composition according to any one of the preceding claims, wherein the at least one amphoteric surfactant is selected from (C8-C20)alkylbetaines, (C8-C20)alkylamido (C1-C6)alkylbetaines, sulfobetaines, (C8-C20)alkylsulfobetaines, (C8-C20)alkylamido(C1-C6)alkylsulfobetaines, (C8-C20)alkylamphoacetate, (C8-C20)alkylamphodiacetate, and their salts, and mixtures thereof

5. The hair cosmetic composition according to any one of the preceding claims, wherein the at least one amphoteric surfactant is selected from coco-betaine, cocamidopropylbetaine, sodium cocoamphoacetate, disodium cocoamphodiacetate, and mixtures thereof.

6. The hair cosmetic composition according to any one of the preceding claims, wherein the amount of amphoteric surfactants ranges from about 0.1% to about 10% by weight, preferably from about 0.5% to about 8% by weight, more preferably from about 0.8% to about 6% by weight, and even more preferably from about 1% to about 5% by weight, relative to the total weight of the composition.

7. The hair cosmetic composition according to any one of the preceding claims, wherein the at least one thiol-based compound is selected from thiolactic acid.

8. The hair cosmetic composition according to any one of the preceding claims, wherein the at least one thiol-based compound is present in an amount of from about 0.5% to about 15% by weight, preferably from about 0.6% to about 14% by weight, more preferably from about 0.7% to about 12% by weight, even more preferably from about 0.8% to about 10% by weight or from about 0.8% to about 10% by weight, based on the total weight of the composition.

9. The hair cosmetic composition according to any one of the preceding claims, wherein the at least one thickening agent is selected from cellulose polymers, gums, modified or unmodified carboxyvinyl polymers, polyacrylamides, copolymers of acrylic acid and of acrylamide, sodium salts of polyhydroxycarboxylic acids, optionally crosslinked and/or neutralized 2-acrylamido-2-methylpropanesulphonic acid polymers and copolymers, polyacrylic acid/alkyl acrylate, glucans, modified or unmodified starches, silicas, and mixtures thereof.

10. The hair cosmetic composition according to any one of the preceding claims, wherein the at least one thickening agent is present in an amount of from about 0.01% to about 5% by weight, preferably from about 0.05% to about 3% by weight, and most preferably from about 0.1% to about 2% by weight, relative to the total weight of the composition.

11. The hair cosmetic composition according to any one of the preceding claims, wherein the at least one nonionic surfactant is selected from fatty alcohols, alkoxylated fatty alcohols, alkyl(ether)phosphates, alkylpolyglucosides, fatty acid alkanolamides, and mixtures thereof.

12. The hair cosmetic composition according to any one of the preceding claims, wherein the at least one nonionic surfactant is present in an amount of from about 0.01% to about 15% by weight, preferably from about 0.1% to about 12% by weight, or more preferably from about 0.5% to about 10% by weight, or even more preferably from about 1% to about 8% by weight, relative to the total weight of the composition.

13. The hair cosmetic composition according to any one of the preceding claims, wherein the at least one cationic conditioning polymer is selected from cationic cellulose derivatives, cationic gum derivatives, polymer derivatives of diallyldimethyl ammonium chloride, polymer derivatives of methacrylamidopropyltrimethylammonium chloride, and mixtures thereof and is present in an amount of from about 0.01% to about 4% by weight, preferably from about 0.05% to about 3% by weight, and most preferably from about 0.05% to about 2% by weight, relative to the total weight of the composition.

14. The hair cosmetic composition according to the preceding claim, further comprising at least one amino silicone compound selected from amodimethicone, trideceth-9 PG amodimethicone, PEG-40/PPG-8 Methylaminopropyl/hydroxypropyl dimethicone copolymer, and mixtures thereof and is present in an amount of from about 0.01% to about 5% by weight, preferably from about 0.05% to about 3% by weight, and most preferably from about 0.1% to about 2% by weight, relative to the total weight of the composition.

15. The composition, according to any one of the preceding claims, further comprising at least one neutralizing agent wherein the at least one neutralizing agent is selected from organic amines, alkali metal hydroxides, alkali earth metal hydroxides, alkali metal carbonates, alkali metal phosphates, and mixtures thereof, and preferably selected from aminomethyl propanol, sodium hydroxide, potassium hydroxide, lithium hydroxide, aminomethyl propanediol, triisopropanol amine, dimethylstearylamine, dimethyl/tallowamine, lysine, ornithine, arginine, monoethanolamine, triethanolamine, calcium hydroxide, calcium bicarbonate, and mixtures thereof.

16. The composition, according to the preceding claim, wherein the at least neutralizing agent is present in an amount of from 0.01% to 3% by weight, preferably from 0.02% to 2.75% by weight, more preferably from 0.025% to 2.5% by weight, even more preferably from 0.03% to 2% by weight, relative to the total weight of the composition.

17. The composition, according to any one of the preceding claims, wherein the pH of the composition ranges from about pH 2.5 to about 6.5, or from about pH 3 to about 6, or from about pH 3 to about 5.2, or from about pH 3 to about 5, or preferably from about pH 3 to about 4.8, or more preferably from about pH 3 to about 4.5, or even more preferably from about pH 3 to about 4.

18. The hair cosmetic composition according to any one of the preceding claims, comprising:
(a) at least one cationic surfactant selected from quaternary ammonium salts, and preferably from cetrimonium chloride, behentrimonium chloride, and mixtures thereof and present in an amount of from about 0.5% to about 8% by weight;
(b) at least one amphoteric surfactant selected from (C8-C20)alkylbetaines, preferably from coco betaine, cocamidopropylbetaine, cocoamphoacetate, cocoamphodiacetate, and their salts, and mixtures thereof and present in an amount of from about 0.8% to about 6% by weight;
(c) at least one thiol-based compound selected from thiolactic acid, their salts, and mixtures thereof and present in an amount of from about 0.7% to about 12% by weight;
(d) at least one thickening agent selected from hydroxyethylcellulose (also known as HEC), hydroxymethylcellulose, methylhydroxyethylcellulose, hydroxypropylcellulose (also known as HPC), hydroxybutylcellulose, hydroxyethylmethylcellulose (also known as methyl hydroxyethylcellulose) and hydroxypropylmethylcellulose (also known as HPMC), cetyl hydroxyethylcellulose, hydroxypropyl guar gums, acrylate/C10 C30 alkyl acrylate copolymers, carbomers, polyacrylates, potato starch modified, and mixtures thereof and present in an amount of from about 0.05% to about 1% by weight;
(e) at least one nonionic surfactant selected from fatty alcohols, alkoxylated fatty alcohols, alkyl(ether)phosphates, alkylpolyglucosides, fatty acid alkanolamides, and mixtures thereof, and present in an amount of from about 0.05% to about 10% by weight;
(f) at least one cationic conditioning polymer selected from cationic cellulose derivatives, cationic gum derivatives, polymer derivatives of diallyldimethyl ammonium chloride, polymer derivatives of methacrylamidopropyltrimethylammonium chloride, and mixtures thereof, and preferably from polyquaternium-7, polyquaternium-10, guar hydroxypropyltrimonium chloride, and mixtures thereof, and present in an amount of from about 0.05% to about 3% by weight;
(g) optionally, at least one amino silicone selected from amodimethicone, trideceth-9 PG amodimethicone, PEG-40/PPG-8 Methylaminopropyl/hydroxypropyl dimethicone copolymer, and mixtures thereof and present in an amount of from about 0.01% to about 5% by weight;
(h) water; and
(i) optionally, at least one neutralizing agent;
wherein the pH of the composition ranges from about 3 to about 5;
all weights being relative to the total weight of the composition.

19. The hair cosmetic composition according to any one of the preceding claims, wherein the composition is substantially free of anionic surfactants selected from sulfate surfactants, sulfonate surfactants, sarcosinate surfactants, and mixtures thereof.

20. The hair cosmetic composition according to any one of the preceding claims, wherein the hair cosmetic composition is a cleansing or shampoo composition.

21. A method of treating hair, the method comprising a treatment cycle involving the steps of:
a) optionally, washing/rinsing the hair with a shampoo having a neutral pH and/or rinsing the hair with water, followed by allowing the hair to air dry or by drying the hair at a temperature ranging from room temperature up to about 100°C, while optionally applying a smoothing action on the hair;
b) applying the composition as defined in any one of the preceding claims onto the hair;
c) allowing the composition in b) to remain on the hair for a period of time ranging from about 1 to about 10 minutes or from about 1 to about 5 minutes;
d) rinsing the hair with water;
e) drying the hair at a temperature ranging from room temperature up to about 100°C, while optionally applying a smoothing action on the hair;
f) passing a flat iron over the hair swatch at least once; and
g) washing/rinsing the hair with a shampoo having a neutral pH and/or rinsing the hair with water, followed by allowing the hair to air dry, while optionally applying a smoothing action on the hair.

22. The method of the preceding claim, wherein the flat iron is employed at a temperature ranging from 100°C to 250°C or from 110°C to 230°C or from 110°C to 210°C or from 120°C to 200°C or from 150°C to 190°C, or from 190°C to 230°C, and preferably, at 230°C.

23. The method according to claim 21 or claim 22, wherein the flat iron is passed over the hair for two times up to 10 times.

24. The method according to any one of claims 21 to 23, wherein the treatment cycle is repeated over a period of days.

25. A method for imparting to hair one or more hair care effects of:
(i) cleansing;
(ii) conditioning;
(iii) straightening effects;
(iv) manageability;
(v) frizz control;
(vi) volume reduction or volume control;
(vii) styling or shaping effects;
(viii) curling effects;
(ix) texlaxing effects;
(x) improvement or retention of curl definition;
(xi) humidity resistance;
(xii) cosmeticity to the feel;
(xiii) smooth feel;
(xiv) natural feel;
(xv) less or reduced rough ends; and/or
(xvi) improvement of the appearance of hair;
comprising applying a hair cosmetic composition of claim 1 or claim 18 to the hair.

26. The method of the preceding claim comprising applying the hair cosmetic composition to hair more than once over a period of days.

## Patentansprüche

1. Haarkosmetische Zusammensetzung, die Folgendes umfasst:
(a) mindestens ein kationisches Tensid;
(b) mindestens ein amphoteres Tensid;
(c) mindestens eine Thiol-basierte Verbindung, die aus Thiomilchsäure, ihren Salzen und Mischungen davon ausgewählt ist;
(d) mindestens ein Verdickungsmittel;
(e) mindestens ein nichtionisches Tensid;
(f) mindestens ein kationisches Konditionierungspolymer;
(g) optional mindestens ein Aminosilikon; und
(h) Wasser; und
wobei der pH-Wert der Zusammensetzung im Bereich von 2 bis kleiner als 7 liegt.

2. Haarkosmetische Zusammensetzung nach dem vorhergehenden Anspruch,
wobei das mindestens eine kationische Tensid aus optional polyoxyalkenylierten primären, sekundären, tertiären Fettaminsalzen, quartären Ammoniumsalzen und Mischungen davon und vorzugsweise aus quartären Ammoniumsalzen und mehr bevorzugt aus Cetrimoniumchlorid, Behentrimoniumchlorid, Behentrimoniummethosulfat, Dipalmitoylethyl-Hydroxyethylmethylammonium-Methosulfat und Mischungen davon ausgewählt ist.

3. Haarkosmetische Zusammensetzung nach einem der vorhergehenden Ansprüche, wobei das mindestens eine kationische Tensid in einer Menge von etwa 0,1 bis etwa 10 Gew.-%, vorzugsweise von etwa 0,5 bis etwa 8 Gew.-%, am meisten bevorzugt von etwa 0,7 bis etwa 5 Gew.-% vorliegt, bezogen auf das Gesamtgewicht der Zusammensetzung.

4. Haarkosmetische Zusammensetzung nach einem der vorhergehenden Ansprüche, wobei das mindestens ein amphotere Tensid aus C₈-C₂₀-Alkylbetainen, C₈-C₂₀-Alkylamido(C₁-C₆)alkylbetainen, Sulfobetainen, C₈-C₂₀-Alkylsulfobetainen, C₈-C₂₀-Alkylamido(C₁-C₆)alkylsulfobetainen, C₈-C₂₀-Alkylamphoacetat, C₈-C₂₀-Alkylamphodiacetat, und ihren Salzen, sowie Mischungen davon ausgewählt ist.

5. Haarkosmetische Zusammensetzung nach einem der vorhergehenden Ansprüche, wobei das mindestens ein amphotere Tensid aus Cocobetain, Cocamidopropylbetain, Natriumcocoamphoacetat, Dinatriumcocoamphodiacetat und Mischungen davon ausgewählt ist.

6. Haarkosmetische Zusammensetzung nach einem der vorhergehenden Ansprüche, wobei die Menge an amphoteren Tensiden im Bereich von etwa 0,1 bis etwa 10 Gew.-%, vorzugsweise von etwa 0,5 bis etwa 8 Gew.-%, mehr bevorzugt von etwa 0,8 bis etwa 6 Gew.-%, und noch mehr bevorzugt von etwa 1 bis etwa 5 Gew.-% vorliegt, bezogen auf das Gesamtgewicht der Zusammensetzung.

7. Haarkosmetische Zusammensetzung nach einem der vorhergehenden Ansprüche, wobei die mindestens eine Thiol-basierte Verbindung aus Thiomilchsäure ausgewählt ist.

8. Haarkosmetische Zusammensetzung nach einem der vorhergehenden Ansprüche, wobei die mindestens eine Thiol-basierte Verbindung in einer Menge von etwa 0,5 bis etwa 15 Gew.-%, vorzugsweise von etwa 0,6 bis etwa 14 Gew.-%, mehr bevorzugt von etwa 0,7 bis etwa 12 Gew.-%, sogar noch mehr bevorzugt von etwa 0,8 bis etwa 10 Gew.-% oder von etwa 0,8 bis etwa 10 Gew.-% vorliegt, bezogen auf das Gesamtgewicht der Zusammensetzung.

9. Haarkosmetische Zusammensetzung nach einem der vorhergehenden Ansprüche, wobei das mindestens eine Verdickungsmittel aus Cellulosepolymeren, Gummis, modifizierten oder unmodifizierten Carboxyvinylpolymeren, Polyacrylamiden, Copolymeren von Acrylsäure und von Acrylamid, Natriumsalzen von Polyhydroxycarbonsäuren ausgewählt ist, optional aus vernetzten und/oder neutralisierten 2-Acrylamido-2-methylpropansulfonsäure-Polymeren- und -Copolymeren, Polyacrylsäure/Alkylacrylat, Glucanen, modifizierten oder unmodifizierten Stärken, Kieselsäuren und Mischungen davon.

10. Haarkosmetische Zusammensetzung nach einem der vorhergehenden Ansprüche, wobei das mindestens eine Verdickungsmittel in einer Menge von etwa 0,01 bis etwa 5 Gew.-%, vorzugsweise von etwa 0,05 bis etwa 3 Gew.-%, und am meisten bevorzugt von etwa 0,1 bis etwa 2 Gew.-% vorliegt, bezogen auf das Gesamtgewicht der Zusammensetzung.

11. Haarkosmetische Zusammensetzung nach einem der vorhergehenden Ansprüche, wobei das mindestens ein nichtionische Tensid aus Fettalkoholen, alkoxylierten Fettalkoholen, Alkyl(ether)phosphaten, Alkylpolyglucosiden, Fettsäurealkanolamiden und Mischungen davon ausgewählt ist.

12. Haarkosmetische Zusammensetzung nach einem der vorhergehenden Ansprüche, wobei das mindestens eine nichtionische Tensid in einer Menge von etwa 0,01 bis etwa 15 Gew.-%, vorzugsweise von etwa 0,1 bis etwa 12 Gew.-%, oder mehr bevorzugt von etwa 0,5 bis etwa 10 Gew.-% oder sogar noch mehr bevorzugt von etwa 1 bis etwa 8 Gew.-% vorliegt, bezogen auf das Gesamtgewicht der Zusammensetzung.

13. Haarkosmetische Zusammensetzung nach einem der vorhergehenden Ansprüche, wobei das mindestens eine kationische Konditionierungspolymer aus kationischen Cellulosederivaten, kationischen Gummiderivaten, Polymerderivaten von Diallyldimethylammoniumchlorid, Polymerderivaten von Methacrylamidopropyltrimethylammoniumchlorid und Mischungen davon ausgewählt ist und in einer Menge von etwa 0,01 bis etwa 4 Gew.-%, vorzugsweise von etwa 0,05 bis etwa 3 Gew.-%, und am meisten bevorzugt von etwa 0,05 bis etwa 2 Gew.-% vorliegt, bezogen auf das Gesamtgewicht der Zusammensetzung.

14. Haarkosmetische Zusammensetzung nach einem der vorhergehenden Ansprüche, ferner umfassend mindestens eine Aminosiliconverbindung, die aus Amodimethicon, Trideceth-9-PG-Amodimethicon, PEG-40/PPG-8 Methylaminopropyl- /Hydroxypropyldimethicon-Copolymer und Mischungen davon ausgewählt ist und in einer Menge von etwa 0,01 bis etwa 5 Gew.-%, vorzugsweise von etwa 0,05 bis etwa 3 Gew.-%, und am meisten bevorzugt von etwa 0,1 bis etwa 2 Gew.-% vorliegt, bezogen auf das Gesamtgewicht der Zusammensetzung.

15. Zusammensetzung nach einem der vorhergehenden Ansprüche, die ferner mindestens ein Neutralisationsmittel umfasst, wobei das mindestens eine Neutralisationsmittel aus organischen Aminen, Alkalimetallhydroxiden, Erdalkalimetallhydroxiden, Alkalimetallcarbonaten, Alkalimetallphosphaten und Mischungen davon ausgewählt ist, und vorzugsweise ausgewählt ist aus Aminomethylpropanol, Natriumhydroxid, Kaliumhydroxid, Lithiumhydroxid, Aminomethylpropandiol, Triisopropanolamin, Dimethylstearylamin, Dimethyl- /Talgamin, Lysin, Ornithin, Arginin, Monoethanolamin, Triethanolamin, Calciumhydroxid, Calciumbicarbonat und Mischungen davon.

16. Zusammensetzung nach einem der vorhergehenden Ansprüche, wobei das mindestens eine Neutralisationsmittel in einer Menge von 0,01 bis 3 Gew.-%, vorzugsweise von 0,02 bis 2,75 Gew.-%, mehr bevorzugt von 0,025 bis 2,5 Gew.-%, noch mehr bevorzugt von 0,03 bis 2 Gew.-% vorliegt, bezogen auf das Gesamtgewicht der Zusammensetzung.

17. Zusammensetzung nach einem der vorhergehenden Ansprüche, wobei der pH-Wert der Zusammensetzung im Bereich von etwa pH 2,5 bis etwa 6,5 oder von etwa pH 3 bis etwa 6 oder von etwa pH 3 bis etwa 5.2 oder von etwa pH 3 bis etwa 5 liegt, oder vorzugsweise im Bereich von etwa pH 3 bis etwa 4,8, oder mehr bevorzugt von etwa pH 3 bis etwa 4,5, oder sogar noch mehr bevorzugt von etwa pH 3 bis etwa 4.

18. Haarkosmetische Zusammensetzung nach einem der vorhergehenden Ansprüche, die Folgendes umfasst:
(a) mindestens ein kationisches Tensid, das aus quartären Ammoniumsalzen und vorzugsweise aus Cetrimoniumchlorid, Behentrimoniumchlorid und Mischungen davon ausgewählt ist und in einer Menge von etwa 0,5 bis etwa 8 Gew.-% vorliegt;
b) mindestens ein amphoteres Tensid, das aus C₈-C₂₀-Alkylbetainen, vorzugsweise aus Cocobetain, Cocamidopropylbetain, Cocoamphoacetat, Cocoamphodiacetat und ihren Salzen sowie Mischungen davon ausgewählt ist und in einer Menge von etwa 0,8 bis etwa 6 Gew.-% vorliegt;
c) mindestens eine Thiol-basierte Verbindung, die aus Thiomilchsäure, ihren Salzen und Mischungen davon ausgewählt ist und in einer Menge von etwa 0,7 bis etwa 12 Gew.-% vorliegt;
d) mindestens ein Verdickungsmittel, das aus Hydroxyethylcellulose (auch bekannt als HEC), Hydroxymethylcellulose, Methylhydroxyethylcellulose, Hydroxypropylcellulose (auch bekannt als HPC), Hydroxybutylcellulose, Hydroxyethylmethylcellulose (auch bekannt als Methylhydroxyethylcellulose) und Hydroxypropylmethylcellulose (auch bekannt als HPMC), Cetylhydroxyethylcellulose, Hydroxypropylguargummis, Acrylat/C₁₀-C₃₀-Alkylacrylat-Copolymeren, Carbomeren, Polyacrylaten, modifizierter Kartoffelstärke und Mischungen davon ausgewählt ist und in einer Menge von etwa 0,05 bis etwa 1 Gew.-% vorliegt;
(e) mindestens ein nichtionisches Tensid, das aus Fettalkoholen, alkoxylierten Fettalkoholen, Alkyl(ether)phosphaten, Alkylpolyglucosiden, Fettsäuralkanolamiden und Mischungen davon ausgewählt ist und in einer Menge von etwa 0,05 bis etwa 10 Gew.-% vorliegt;
f) mindestens ein kationisches Konditionierungspolymer, das aus kationischen Cellulosederivaten, kationischen Gummiderivaten, Polymerderivaten von Diallyldimethylammoniumchlorid, Polymerderivaten von Methacrylamidopropyltrimethylammoniumchlorid und Mischungen davon ausgewählt ist, und vorzugsweise aus Polyquaternium-7, Polyquaternium-10, Guarhydroxypropyltrimoniumchlorid und Mischungen davon, und in einer Menge von etwa 0,05 bis etwa 3 Gew.-% vorliegt;
(g) optional mindestens ein Aminosilikon, das aus Amodimethicon, Trideceth-9-PG-amodimethicon, PEG-40/PPG-8-Methylaminopropyl- /Hydroxypropyldimethicon-Copolymer und Mischungen davon ausgewählt ist und in einer Menge von etwa 0,01 bis etwa 5 Gew.-% vorliegt;
(h) Wasser; und
(i) optional mindestens ein Neutralisationsmittel;
wobei der pH-Wert der Zusammensetzung im Bereich von etwa 3 bis etwa 5 liegt; wobei alle Gewichte auf das Gesamtgewicht der Zusammensetzung bezogen sind.

19. Haarkosmetische Zusammensetzung nach einem der vorhergehenden Ansprüche, wobei die Zusammensetzung im Wesentlichen frei ist von anionischen Tensiden, die aus Sulfattensiden, Sulfonattensiden, Sarcosinattensiden und Mischungen davon ausgewählt sind.

20. Haarkosmetische Zusammensetzung nach einem der vorhergehenden Ansprüche, wobei die haarkosmetische Zusammensetzung eine Reinigungs- oder Shampoozusammensetzung ist.

21. Verfahren zur Haarbehandlung, wobei das Verfahren einen Behandlungszyklus mit den folgenden Schritten umfasst:
a) optional Waschen/Spülen der Haare mit einem Shampoo mit einem neutralen pH-Wert und/oder Spülen der Haare mit Wasser, wobei man die Haare anschließend lufttrocknen lässt oder die Haare bei einer Temperatur im Bereich von Raumtemperatur bis etwa 100°C trocknet, während optional eine glättende Maßnahme an dem Haar durchgeführt wird;
b) Aufbringen der Zusammensetzung nach einem der vorhergehenden Ansprüche auf das Haar;
c) die Zusammensetzung in Schritt b) für einen Zeitraum im Bereich von etwa 1 Minute bis etwa 10 Minuten oder von etwa 1 Minute bis etwa 5 Minuten auf dem Haar belassen;
d) optional Spülen der Haare mit Wasser;
e) Trocknen der Haare bei einer Temperatur im Bereich von Raumtemperatur bis etwa 100°C, während optional eine glättende Maßnahme an dem Haar durchgeführt wird;
f) ein Glätteisen mindestens einmal über die Haarsträhnen führen; und
g) Waschen/Spülen der Haare mit einem Shampoo mit einem neutralen pH-Wert und/oder Spülen der Haare mit Wasser, wobei man die Haare anschließend lufttrocknen lässt, während optional eine glättende Maßnahme an dem Haar durchgeführt wird.

22. Verfahren nach dem vorhergehenden Anspruch, wobei das Glätteisen bei einer Temperatur im Bereich von 100°C bis 250°C oder von 110°C bis 230°C oder von 110°C bis 210°C oder von 120°C bis 200°C oder von 150°C bis 190°C oder von 190°C bis 230°C und vorzugsweise bei 230°C verwendet wird.

23. Verfahren nach Anspruch 21 oder Anspruch 22, wobei das Glätteisen zwei- bis 10-mal über das Haar geführt wird.

24. Verfahren nach einem der Ansprüche 21 bis 23, wobei der Behandlungszyklus über mehrere Tage wiederholt wird.

25. Verfahren zur Anwendung einer oder mehrerer der folgenden Haarpflegemaßnahmen auf das Haar:
(i) Reinigung;
(ii) Konditionierung;
(iii) Glättungseffekte;
(iv) Kämmbarkeit;
(v) Eindämmung des Kräuseins;
(iv) Volumenverringerung oder Volumenkontrolle;
(vii) Styling- oder Formungseffekte;
(viii) Lockenbildung;
(ix) Texlaxing-Effekte;
(x) Verbesserung oder Beibehaltung der Ausprägung von Locken;
(xi) Feuchtigkeitsbeständigkeit;
(xii) Pflege für mehr Geschmeidigkeit;
(xiii) glattes Gefühl;
(xiv) natürliches Gefühl;
(xv) weniger oder reduzierte raue Enden; und/oder
(xvi) Verbesserung des Erscheinungsbildes der Haare;
umfassend das Aufbringen einer haarkosmetischen Zusammensetzung nach Anspruch 1 oder Anspruch 18 auf das Haar.

26. Verfahren nach dem vorhergehenden Anspruch, bei dem die haarkosmetische Zusammensetzung mehr als einmal über mehrere Tage auf das Haar aufgebracht wird.

## Revendications

1. Composition cosmétique capillaire comprenant :
(a) au moins un tensioactif cationique ;
(b) au moins un tensioactif amphotère ;
(c) au moins un composé à base de thiol sélectionné parmi un acide thiolactique, leurs sels, et les mélanges de ceux-ci ;
(d) au moins un agent épaississant ;
(e) au moins un tensioactif non ionique ;
(f) au moins un polymère cationique de conditionnement ;
(g) facultativement, au moins une silicone aminée ; et
(h) de l'eau ; et
dans laquelle le pH de la composition est de 2 à moins de 7.

2. Composition cosmétique capillaire selon la revendication précédente, dans laquelle l'au moins un tensioactif cationique est sélectionné parmi les sels d'amine grasse primaire, secondaire, tertiaire, facultativement polyoxyalcénylée, les sels d'ammonium quaternaire, et les mélanges de ceux-ci, et de préférence parmi les sels d'ammonium quaternaire, et de manière davantage préférée parmi le chlorure de cétrimonium, le chlorure de béhentrimonium, le méthosulfate de béhentrimonium, le méthosulfate de dipalmitoyléthyl-hydroxyéthylméthylammonium, et les mélanges de ceux-ci.

3. Composition cosmétique capillaire selon l'une quelconque des revendications précédentes, dans laquelle l'au moins un tensioactif cationique est présent en une quantité d'environ 0,1 % à environ 10 % en poids, de préférence d'environ 0,5 % à environ 8 % en poids, et de manière préférée entre toutes d'environ 0,7 % à environ 5 % en poids, par rapport au poids total de la composition.

4. Composition cosmétique capillaire selon l'une quelconque des revendications précédentes, dans laquelle l'au moins un tensioactif amphotère est sélectionné parmi les (alkyle en C8-C20)bétaïnes, les (alkyle en C8-C20)-amido-(alkyle en C1-C6)bétaïnes, les sulfobétaïnes, les (alkyle en C8-C20)sulfobétaïnes, les (alkyle en C8-C20)-amido-(alkyle en C1-C6)sulfobétaïnes, un amphoacétate d'alkyle en C8-C20, un amphodiacétate d'alkyle en C8-C20, et leurs sels, et les mélanges de ceux-ci.

5. Composition cosmétique capillaire selon l'une quelconque des revendications précédentes, dans laquelle l'au moins un tensioactif amphotère est sélectionné parmi la cocobétaïne, la cocamidopropylbétaïne, le cocoamphoacétate de sodium, le cocoamphodiacétate de disodium et les mélanges de ceux-ci.

6. Composition cosmétique capillaire selon l'une quelconque des revendications précédentes, dans laquelle la quantité de tensioactifs amphotères est d'environ 0,1 % à environ 10 % en poids, de préférence d'environ 0,5 % à environ 8 % en poids, de manière davantage préférée d'environ 0,8 % à environ 6 % en poids, et de manière encore plus préférée d'environ 1 % à environ 5 % en poids, par rapport au poids total de la composition.

7. Composition cosmétique capillaire selon l'une quelconque des revendications précédentes, dans laquelle l'au moins un composé à base de thiol est sélectionné parmi un acide thiolactique.

8. Composition cosmétique capillaire selon l'une quelconque des revendications précédentes, dans laquelle l'au moins un composé à base de thiol est présent en une quantité d'environ 0,5 % à environ 15 % en poids, de préférence d'environ 0,6 % à environ 14 % en poids, de manière davantage préférée d'environ 0,7 % à environ 12 % en poids, de manière encore plus préférée d'environ 0,8 % à environ 10 % en poids ou d'environ 0,8 % à environ 10 % en poids, sur la base du poids total de la composition.

9. Composition cosmétique capillaire selon l'une quelconque des revendications précédentes, dans laquelle l'au moins un agent épaississant est sélectionné parmi les polymères de cellulose, les gommes, les polymères carboxyvinyliques modifiés ou non modifiés, les polyacrylamides, les copolymères d'acide acrylique et d'acrylamide, les sels de sodium d'acides polyhydroxycarboxyliques, les polymères et copolymères d'acide 2-acrylamido-2-méthylpropanesulfonique facultativement réticulés et/ou neutralisés, un polyacide acrylique/acrylate d'alkyle, les glucanes, les amidons modifiés ou non modifiés, les silices, et les mélanges de ceux-ci.

10. Composition cosmétique capillaire selon l'une quelconque des revendications précédentes, dans laquelle l'au moins un agent épaississant est présent en une quantité d'environ 0,01 % à environ 5 % en poids, de préférence d'environ 0,05 % à environ 3 % en poids, et de manière préférée entre toutes d'environ 0,1 % à environ 2 % en poids, par rapport au poids total de la composition.

11. Composition cosmétique capillaire selon l'une quelconque des revendications précédentes, dans laquelle l'au moins un tensioactif non ionique est sélectionné parmi les alcools gras, les alcools gras alcoxylés, les alkyl-(éther)phosphates, les alkylpolyglucosides, les alcanolamides d'acide gras, et les mélanges de ceux-ci.

12. Composition cosmétique capillaire selon l'une quelconque des revendications précédentes, dans laquelle l'au moins un tensioactif non ionique est présent en une quantité d'environ 0,01 % à environ 15 % en poids, de préférence d'environ 0,1 % à environ 12 % en poids, ou de manière davantage préférée d'environ 0,5 % à environ 10 % en poids, ou de manière encore plus préférée d'environ 1 % à environ 8 % en poids, par rapport au poids total de la composition.

13. Composition cosmétique capillaire selon l'une quelconque des revendications précédentes, dans laquelle l'au moins un polymère cationique de conditionnement est sélectionné parmi les dérivés cellulosiques cationiques, les dérivés cationiques de gomme, les dérivés polymères de chlorure de diallyldiméthylammonium, les dérivés polymères de chlorure de méthacrylamidopropyltriméthylammonium, et les mélanges de ceux-ci et est présent en une quantité d'environ 0,01 % à environ 4 % en poids, de préférence d'environ 0,05 % à environ 3 % en poids, et de manière préférée entre toutes d'environ 0,05 % à environ 2 % en poids, par rapport au poids total de la composition.

14. Composition cosmétique capillaire selon la revendication précédente, comprenant en outre au moins un composé de silicone aminée sélectionné parmi l'amodiméthicone, la tridéceth-9 PG amodiméthicone, un copolymère de méthylaminopropyl/hydroxypropyl diméthicone PEG-40/PPG-8, et les mélanges de ceux-ci et est présent en une quantité d'environ 0,01 % à environ 5 % en poids, de préférence d'environ 0,05 % à environ 3 % en poids, et de manière préférée entre toutes d'environ 0,1 % à environ 2 % en poids, par rapport au poids total de la composition.

15. Composition selon l'une quelconque des revendications précédentes, comprenant en outre au moins un agent de neutralisation, dans laquelle l'au moins un agent de neutralisation est sélectionné parmi les amines organiques, les hydroxydes de métal alcalin, les hydroxydes de métal alcalino-terreux, les carbonates de métal alcalin, les phosphates de métal alcalin, et les mélanges de ceux-ci, et de préférence sélectionné parmi l'aminométhylpropanol, l'hydroxyde de sodium, l'hydroxyde de potassium, l'hydroxyde de lithium, l'aminométhylpropanediol, la triisopropanolamine, la diméthylstéarylamine, la diméthyl/suifamine, la lysine, l'ornithine, l'arginine, la monoéthanolamine, la triéthanolamine, l'hydroxyde de calcium, le bicarbonate de calcium, et les mélanges de ceux-ci.

16. Composition selon la revendication précédente, dans laquelle l'au moins un agent de neutralisation est présent en une quantité de 0,01 % à 3 % en poids, de préférence de 0,02 % à 2,75 % en poids, de manière davantage préférée de 0,025 % à 2,5 % en poids, de manière encore plus préférée de 0,03 % à 2 % en poids, par rapport au poids total de la composition.

17. Composition selon l'une quelconque des revendications précédentes, dans laquelle le pH de la composition est d'environ pH 2,5 à environ 6,5, ou d'environ pH 3 à environ 6, ou d'environ pH 3 à environ 5,2, ou d'environ pH 3 à environ 5, ou de préférence d'environ pH 3 à environ 4,8, ou de manière davantage préférée d'environ pH 3 à environ 4,5, ou de manière encore plus préférée d'environ pH 3 à environ 4.

18. Composition cosmétique capillaire selon l'une quelconque des revendications précédentes, comprenant :
(a) au moins un tensioactif cationique sélectionné parmi les sels d'ammonium quaternaire, et de préférence parmi le chlorure de cétrimonium, le chlorure de béhentrimonium, et les mélanges de ceux-ci et présent en une quantité d'environ 0,5 % à environ 8 % en poids ;
(b) au moins un tensioactif amphotère sélectionné parmi les (alkyle en C8-C20)bétaïnes, de préférence parmi la cocobétaïne, la cocamidopropylbétaïne, un cocoamphoacétate, un cocoamphodiacétate, et leurs sels, et les mélanges de ceux-ci et présent en une quantité d'environ 0,8 % à environ 6 % en poids ;
(c) au moins un composé à base de thiol sélectionné parmi un acide thiolactique, leurs sels, et les mélanges de ceux-ci et présent en une quantité d'environ 0,7 % à environ 12 % en poids ;
(d) au moins un agent épaississant sélectionné parmi l'hydroxyéthylcellulose (également appelée HEC), l'hydroxyméthylcellulose, la méthylhydroxyéthylcellulose, l'hydroxypropylcellulose (également appelée HPC), l'hydroxybutylcellulose, l'hydroxyéthylméthylcellulose (également appelée méthylhydroxyéthylcellulose) et l'hydroxypropylméthylcellulose (également appelée HPMC), la cétylhydroxyéthylcellulose, les gommes de guar hydroxypropylées, les copolymères d'acrylate/acrylate d'alkyle en C10-C30, les carbomères, les polyacrylates, un amidon de pomme de terre modifié, et les mélanges de ceux-ci et présent en une quantité d'environ 0,05 % à environ 1 % en poids ;
(e) au moins un tensioactif non ionique sélectionné parmi les alcools gras, les alcools gras alcoxylés, les alkyl-(éther)phosphates, les alkylpolyglucosides, les alcanolamides d'acide gras, et les mélanges de ceux-ci, et présent en une quantité d'environ 0,05 % à environ 10 % en poids ;
(f) au moins un polymère cationique de conditionnement sélectionné parmi les dérivés cellulosiques cationiques, les dérivés cationiques de gomme, les dérivés polymères de chlorure de diallyldiméthylammonium, les dérivés polymères de chlorure de méthacrylamidopropyltriméthylammonium, et les mélanges de ceux-ci, et de préférence parmi le polyquaternium-7, le polyquaternium-10, le chlorure d'hydroxypropyltrimonium de guar, et les mélanges de ceux-ci, et présent en une quantité d'environ 0,05 % à environ 3 % en poids ;
(g) facultativement, au moins une silicone aminée sélectionnée parmi l'amodiméthicone, la tridéceth-9 PG amodiméthicone, un copolymère de méthylaminopropyl/hydroxypropyl diméthicone PEG-40/PPG-8, et les mélanges de ceux-ci et présent en une quantité d'environ 0,01 % à environ 5 % en poids ;
(h) de l'eau ; et
(i) facultativement, au moins un agent de neutralisation ; dans laquelle le pH de la composition est d'environ 3 à environ 5 ;
tous les poids étant par rapport au poids total de la composition.

19. Composition cosmétique capillaire selon l'une quelconque des revendications précédentes, dans laquelle la composition est sensiblement exempte de tensioactifs anioniques sélectionnés parmi les tensioactifs à base de sulfate, les tensioactifs à base de sulfonate, les tensioactifs à base de sarcosinate, et les mélanges de ceux-ci.

20. Composition cosmétique capillaire selon l'une quelconque des revendications précédentes, dans laquelle la composition cosmétique capillaire est une composition de nettoyage ou de shampooing.

21. Procédé de traitement des cheveux, le procédé comprenant un cycle de traitement impliquant les étapes suivantes :
a) facultativement, le lavage/rinçage des cheveux avec un shampooing ayant un pH neutre et/ou le rinçage des cheveux avec de l'eau, suivi par le fait de laisser les cheveux sécher à l'air ou de sécher les cheveux à une température allant de la température ambiante jusqu'à environ 100 °C, tout en appliquant facultativement une action de lissage sur les cheveux ;
b) l'application de la composition telle que définie dans l'une quelconque des revendications précédentes sur les cheveux ;
c) le fait de laisser la composition de b) rester sur les cheveux pendant une période de temps allant d'environ 1 à environ 10 minutes ou d'environ 1 à environ 5 minutes ;
d) le rinçage des cheveux avec de l'eau ;
e) le séchage des cheveux à une température allant de la température ambiante jusqu'à environ 100 °C, tout en appliquant facultativement une action de lissage sur les cheveux ;
f) le passage d'un fer plat sur l'échantillon de cheveux au moins une fois ; et
g) le lavage/rinçage des cheveux avec un shampooing ayant un pH neutre et/ou le rinçage des cheveux avec de l'eau, suivi par le fait de laisser les cheveux sécher à l'air, tout en appliquant facultativement une action de lissage sur les cheveux.

22. Procédé selon la revendication précédente, dans lequel le fer plat est utilisé à une température de 100 °C à 250 °C ou de 110 °C à 230 °C ou de 110 °C à 210 °C ou de 120 °C à 200 °C ou de 150 °C à 190 °C, ou de 190 °C à 230 °C, et de préférence, à 230 °C.

23. Procédé selon la revendication 21 ou la revendication 22, dans lequel le fer plat est passé sur les cheveux deux fois jusqu'à 10 fois.

24. Procédé selon l'une quelconque des revendications 21 à 23, dans lequel le cycle de traitement est répété sur une période de plusieurs jours.

25. Procédé pour conférer aux cheveux un ou plusieurs effets de soin capillaire de :
(i) nettoyage ;
(ii) conditionnement ;
(iii) effets de lissage ;
(iv) maniabilité ;
(v) contrôle des frisottis ;
(vi) réduction de volume ou contrôle de volume ;
(vii) effets de coiffage ou de mise en forme ;
(viii) effets de bouclage ;
(ix) effets de texlaxage ;
(x) amélioration ou conservation de la définition d'un frisage ;
(xi) résistance à l'humidité ;
(xii) cosméticité au toucher ;
(xiii) toucher doux ;
(xiv) toucher naturel ;
(XV) extrémités rugueuses moindres ou réduites ; et/ou
(xvi) amélioration de l'apparence des cheveux ;
comprenant l'application d'une composition cosmétique capillaire selon la revendication 1 ou la revendication 18 sur les cheveux.

26. Procédé selon la revendication précédente comprenant l'application de la composition cosmétique capillaire sur les cheveux plus d'une fois sur une période de plusieurs jours.
